(19) Europäisches Patentamt
European Patent Office
Office européen des brevets

(11) Veröffentlichungsnummer: **0 201 459 A1**

(12) # EUROPÄISCHE PATENTANMELDUNG

(21) Anmeldenummer: **86810193.2**

(22) Anmeldetag: **30.04.86**

(51) Int. Cl.[4]: **C 07 D 499/00,** A 61 K 31/43 // C07D205/08, C07F7/18, C07F9/65

(30) Priorität: **06.05.85 CH 1900/85**

(43) Veröffentlichungstag der Anmeldung: **12.11.86 Patentblatt 86/46**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Lang, Marc, Dr., Rue des Ormes 6, F-68170 Rixheim (FR)**

(54) **Acylaminomethylpenemverbindungen, ihre Herstellung und sie enthaltende pharmazeutische Präparate.**

(57) Verbindungen der Formel

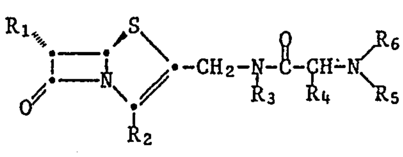

(I)

worin $R_1$ durch Hydroxy oder geschütztes Hydroxy substituiertes Niederalkyl ist, $R_2$ Carboxyl oder funktionell abgewandeltes Carboxyl ist, $R_3$ Wasserstoff oder Niederalkyl darstellt, $R_4$ für Wasserstoff oder für einen über ein Kohlenstoffatom gebundenen organischen Rest steht, $R_5$ Wasserstoff oder Niederalkyl bedeutet, oder zusammen mit $R_4$ gegebenenfalls durch Hydroxy oder Oxo substituiertes Niederalkylen bedeutet, und $R_6$ Wasserstoff, Niederalkyl oder Acyl ist, optische Isomere von Verbindungen der Formel I, Mischungen dieser optischen Isomere und Salze von solchen Verbindungen der Formel I, die eine salzbildende Gruppe aufweisen, besitzen antibiotische Eigenschaften. Die neuen Verbindungen können z.B. in Form von antibiotisch wirksamen Präparaten zur Behandlung von Infektionskrankheiten verwendet werden. Die neuen Verbindungen können in an sich bekannter Weise hergestellt werden.

EP 0 201 459 A1

86810193.2
0201459



BEZEICHNUNG GEÄNDERT
Siehe Titelseite

CIBA-GEIGY AG
Basel (Schweiz)

4-15334/+

## Acylaminomethyl-Verbindungen

Die vorliegende Erfindung betrifft neue Acylaminomethyl-penem-Verbindungen, Verfahren zu ihrer Herstellung, pharmazeutische Präparate, die solche Verbindungen enthalten und ihre Verwendung zur Herstellung von pharmazeutischen Präparaten oder als pharmakologisch wirksame Verbindungen.

Die Erfindung betrifft insbesondere 2-Acylaminomethyl-2-penem-Verbindungen der Formel

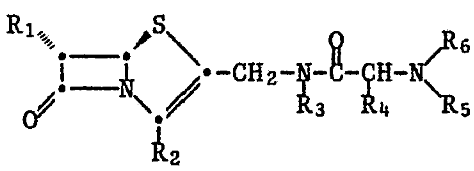

(I),

worin $R_1$ durch Hydroxy oder geschütztes Hydroxy substituiertes Niederalkyl ist, $R_2$ Carboxyl oder funktionell abgewandeltes Carboxyl ist, $R_3$ Wasserstoff oder Niederalkyl darstellt, $R_4$ für Wasserstoff oder für einen über ein Kohlenstoffatom gebundenen organischen Rest steht, $R_5$ Wasserstoff oder Niederalkyl bedeutet, oder zusammen mit $R_4$ gegebenenfalls durch Hydroxy oder Oxo substituiertes Niederalkylen bedeutet, und $R_6$ Wasserstoff, Niederalkyl oder Acyl ist, optische Isomere von Verbindungen der Formel I, Mischungen dieser optischen Isomere und Salze von solchen Verbindungen der Formel I, die eine salzbildende Gruppe aufweisen.

Die vor- und nachstehend verwendeten Definitionen haben im Rahmen der vorliegenden Beschreibung vorzugsweise die folgenden Bedeutungen:

Funktionell abgewandeltes Carboxyl $R_2$ ist insbesondere unter physiologischen Bedingungen spaltbares verestertes Carboxyl oder geschütztes Carboxyl $R_2'$

Eine unter physiologischen Bedingungen spaltbare (metabolisierbare) veresterte Carboxylgruppe $R_2$ ist in erster Linie eine Acyloxymethoxycarbonylgruppe oder auch Acylaminomethoxycarbonylgruppe, worin Acyl z.B. den Rest einer organischen Carbonsäure, in erster Linie einer gegebenenfalls, z.B. durch Amino, substituierten Niederalkancarbonsäure oder Arencarbonsäure, z.B. Benzoesäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, Niederalkanoylaminomethoxycarbonyl, Benzaminomethoxycarbonyl, 4-Crotonolactonyl und 4-Butyrolacton-4-yl. Weitere unter physiologischen Bedingungen spaltbare veresterte Carboxylgruppen $R_2$ sind z.B. 5-Indanyloxycarbonyl, 3-Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes gegebenenfalls durch Niederalkyl oder Phenyl substituiert ist.

Ein über ein Kohlenstoffatom gebundener organischer Rest $R_4$ hat bis zu 18, bevorzugt bis zu 10 Kohlenstoffatome und ist z.B. ein entsprechender gesättigter oder ungesättigter niederaliphatischer, gesättigter oder ungesättigter cycloaliphatischer, aromatischer oder aromatisch-niederaliphatischer Kohlenwasserstoffrest oder ein heterocyclischer oder heterocyclisch-niederaliphatischer Rest.

Ein entsprechender niederaliphatischer Rest $R_4$ ist z.B. Niederalkyl, ferner auch Niederalkenyl.

Ein entsprechender cycloaliphatischer Rest $R_4$ ist beispielsweise Cycloalkyl oder ungesättigtes Cycloalkyl, wie Cycloalkenyl oder Cycloalkadienyl.

Als aromatischer Kohlenwasserstoffrest ist $R_4$ beispielsweise ein entsprechender monocyclischer oder auch polycyclischer, wie bicyclischer Rest, insbesondere Phenyl.

Bei einem aromatisch-niederaliphatischen Kohlenwasserstoffrest $R_4$ handelt es sich beispielsweise um Phenylniederalkyl.

Ein heterocyclischer Rest $R_4$ ist insbesondere ein entsprechender monocyclischer oder polycyclischer, insbesondere monocyclischer oder bicyclischer, ferner auch tricyclischer Rest, wie ein gegebenenfalls partiell gesättigter monocyclischer 5- oder 6-gliedriger Heteroaryl-Rest mit 1 - 4 Ringstickstoffatomen und gegebenenfalls einem zusätzlichen Ringheteroatom ausgewählt aus der Gruppe Sauerstoff und Schwefel, z.B. ein entsprechender aza-, diaza-, triaza-, tetraza-, oxa-, oxaza-, oxadiaza-, oxatriaza-, thia-, thiaza-, thiadiaza- oder thiatriaza-cyclischer Rest aromatischen Charakters oder ein entsprechender Dihydro- oder Tetrahydro-Rest, ferner gegebenenfalls partiell gesättigte Benzo-, Dibenzo-, Pyrido- oder Pyrimidoderivate solcher 5- oder 6-gliedrigen Reste. Entsprechende Heteroarylreste $R_4$ sind beispielsweise Pyrrolyl, wie 2- oder 3-Pyrrolyl, Diazolyl, wie Imidazolyl, z.B. 2-, 4- oder 5-Imidazolyl, oder Pyrazolyl, z.B. 3- oder 4-Pyrazolyl, Triazolyl, z.B. 1,2,3-Triazol-4-yl, 1,2,4-Triazol-5-yl oder 1,2,4-Triazol-3-yl, Tetrazolyl, z.B. 1H-oder 2H-Tetrazol-5-yl, Pyridyl, z.B. 2-, 3- oder 4-Pyridyl, Diazinyl, wie Pyrimidyl, z.B. 2-, 4- oder 5-Pyrimidyl oder Pyrazinyl, z.B. 2-Pyrazinyl, Triazinyl, z.B. 1,2,4-Triazin-6-yl oder 1,3,5-Triazin-2-yl, Furyl, z.B. 2- oder 3-Furyl, Thienyl, z.B. 2- oder 3-Thienyl, Oxazolyl, z.B. 2- oder 4-Oxazolyl, Oxadiazolyl, z.B. 1,2,4-Oxadiazol-3-yl, 1,2,5-Oxadiazol-3-yl, 1,2,4-Oxadiazol-5-yl oder 1,3,4-Oxadiazol-2-yl, Isoxazolyl, z.B. 3-Isoxazolyl, Thiazolyl, z.B. 2- oder 4-Thiazolyl, Thiadiazolyl, z.B. 1,2,4-Thiadiazol-3-yl, 1,2,5-Thiadiazol-3-yl, 1,2,4-Thiadiazol-5-yl oder 1,3,4-Thiadiazol-2-yl, oder Isothiazolyl, z.B. 3- oder 4-Isothiazolyl. Die genannten 5- und 6-gliedrigen Heteroarylreste können auch in partiell gesättigter Form vorliegen. Solche Reste sind beispielsweise Dihydroimidazolyl, z.B. 2,3-Dihydroimidazol-4-yl, Dihydropyridyl, z.B. 1,2-Dihydropyrid-3-yl, Dihydropyrimidyl, z.B. 1,2-Dihydropyrimid-4-yl, Dihydro-und Tetrahydropyrazinyl, z.B. 2,3-Dihydropyrazin-5-yl oder 3,4,5,6-Tetrahydropyrazin-2-yl, oder Dihydro- oder Tetrahydrotriazinyl, z.B. 2,3,4,5-Tetrahydro-1,2,4-triazin-6-yl. Polycyclische Derivate der genannten Heteroarylreste sind z.B. Indol-3-yl, Benzimidazol-2-yl, Benzopyrazol-3-yl, Chinolin-2-yl oder Chinolin-4-yl, Benzothiazol-2-yl, Benzoxazol-2-yl, Pyrido-[2,3-b]pyrid-3-yl oder Pyrido[2,3-b]pyrid-4-yl.

Heterocyclisch-niederaliphatische Reste $R_4$ sind z.B. Niederalkylreste, die durch einen oder zwei, insbesondere einen der obengenannten über ein Kohlenstoffatom gebundenen Heterocyclylreste oder durch einen gegebenenfalls partiell gesättigten monocyclischen, 5- oder 6-gliedrigen, über ein Ringstickstoffatom gebundenen Heteroarylrest, welcher als Ringheteroatome 1 - 4 Stickstoffatome enthält, substituiert sind. Bei den zuletzt genannten über ein Stickstoffatom gebundenen Resten handelt es sich beispielsweise um Imidazol-1-yl, Tetrahydro- oder 4,5-Dihydro-imidazol-1-yl, Pyrazol-1-yl, 1,2,4-Triazol-1-yl, 1,2,3-Triazol-1-yl, Tetrazol-1-yl, Tetrazol-2-yl, 1-Pyridinio, 1,2-Dihydro-1-pyridyl, 1,4-Dihydro-1-pyridyl, 1,2-Dihydro-pyrimid-1-yl oder 1,4-Dihydro-pyrimid-1-yl.

Die genannten organischen Reste $R_4$ sind unsubstituiert oder können z.B. durch gegebenenfalls veräthertes oder verestertes, inklusive geschütztes Hydroxy, z.B. Hydroxy, Niederalkoxy, Phenylniederalkoxy, Niederalkanoyloxy oder Halogen, gegebenenfalls veräthertes Mercapto, z.B. Mercapto, Niederalkylthio oder Phenylthio, Niederalkyl; durch Hydroxy, Niederalkoxy, gegebenenfalls verestertes oder amidiertes Carboxy, wie Carboxy, Niederalkoxycarbonyl oder Carbamoyl, gegebenenfalls N-niederalkyliertes Amino, z.B. Amino, Niederalkylamino oder Diniederalkylamino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Niederalkylen, gegebenenfalls niederalkyliertes Azaniederalkylen, Oxa-, Dioxa- oder Thianiederalkylen, gegebenenfalls substituiertes inklusive geschütztes Amino, z.B. Amino, Niederalkylamino, Diniederalkylamino, Niederalkylenamino, Guanidino, Carbamoylamino, Acylamino, wie Niederalkanoylamino, oder gegebenenfalls durch Amino und/oder Carboxy substituiertes Niederalkoxycarbonylamino, gegebenenfalls funktionell abgewandeltes, inklusive geschütztes Carboxyl oder Sulfo, z.B. Carboxyl, verestertes Carboxyl, wie Niederalkoxycarbonyl, amidiertes Carboxyl, wie gegebenenfalls substituiertes Carbamoyl, z.B. Carbamoyl oder N-mono- oder N,N-diniederalkyliertes Carbamoyl, Cyano, Sulfo oder Sulfamoyl, gegebenenfalls z.B. durch Niederalkyl, Nitro, Amino, Hydroxy, Niederalkoxy, Carboxy und/oder Halogen substituiertes Phenyl, Cycloalkyl, Nitro, Oxo und/oder Oxido substituiert, wie z.B. mono- oder auch poly-, wie insbesondere mono- oder di-, ferner auch trisubstituiert sein.

Eine Acylgruppe $R_6$ hat bis zu 15 Kohlenstoffatome und ist z.B. die Acylgruppe einer Carbonsäure, eines Halbesters der Kohlensäure, einer Carbaminsäure, einer substituierten Carbaminsäure, der Thiocarbaminsäure, einer substituierten Thiocarbaminsäure, einer Sulfonsäure, der Amidosulfonsäure oder einer substituierten Amidosulfonsäure. Solche Reste sind beispielsweise Niederalkanoyl, durch Hydroxy, Niederalkoxy, Amino, Halogen, Carboxy und/oder Cyan substituiertes Niederalkanoyl, Niederalkenoyl, Cycloalkanoyl, Benzoyl, durch Amino, Niederalkylamino, Diniederalkylamino, Niederalkanoylamino, Carboxy, Cyano, Nitro, Halogen und/oder Niederalkyl substituiertes Benzoyl, Phenylniederalkanoyl, im Niederalkanoylteil durch Hydroxy oder Amino substituiertes Phenylniederalkanoyl, Heterocyclylcarbonyl, worin Heterocyclyl einer der bei der Definition der heterocyclischen bzw. heterocyclisch-niederaliphatischen Reste $R_4$ angegebenen gegebenenfalls substituierten, gegebenenfalls partiell gesättigten 5- oder 6-gliedrigen monocyclischen Heteroarylreste ist oder ein gegebenenfalls durch Oxo, Sulfo, Niederalkoxysulfonyl, Sulfamoyl, Niederalkyl und/oder Hydroxy substituierter 5- oder 6-gliedriger Diazacycloalkan-Rest, z.B. Tetrahydro-1-imidazolyl oder Piperazin-1-yl, ist; Niederalkoxycarbonyl, Niederalkoxycarbonyl substituiert durch Carboxy und Amino, Benzyloxycarbonyl, Carbamoyl, Anilinocarbonyl, N-mono- oder N,N-diniederalkyliertes Carbamoyl, Niederalkyl-thiocarbamoyl, Sulfo, Niederalkansulfonyl, durch Halogen substituiertes Niederalkansulfonyl, Benzolsulfonyl, durch Amino, Halogen, Niederalkyl oder Nitro substituiertes Benzolsulfonyl oder Sulfamoyl. Als Acylreste $R_6$ kommen auch die bei den Aminoschutzgruppen genannten Acylreste in Betracht.

In der vorliegenden Beschreibung bedeutet der im Zusammenhang mit Definitionen von Gruppen bzw. Verbindungen verwendete Ausdruck "Nieder", dass die entsprechenden Gruppen bzw. Verbindungen, sofern nicht ausdrücklich anders definiert, 1 bis 7, bevorzugt 1 bis 4 Kohlenstoffatome enthalten.

Durch Hydroxy substituiertes Niederalkyl $R_1$ ist insbesondere in α-Stellung zum Penem-Ringgerüst durch Hydroxy substituiertes Niederalkyl und bedeutet z.B. Hydroxymethyl, 1-Hydroxyäthyl, 1-Hydroxyprop-1-yl, 2-Hydroxyprop-2-yl, 1-Hydroxybut-1-yl oder 2-Hydroxybut-2-yl.

Niederalkanoyloxymethoxycarbonyl ist z.B. Acetoxymethoxycarbonyl oder Pivaloyloxymethoxycarbonyl.

α-Aminoniederalkanoyloxymethoxycarbonyl ist z.B. Glycyloxymethoxycarbonyl, L-Valyloxymethoxycarbonyl oder L-Leucyloxymethoxycarbonyl.

Niederalkanoylaminomethoxycarbonyl ist z.B. Acetaminomethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche in 5-Stellung des Dioxolen-Ringes gegebenenfalls durch Niederalkyl oder Phenyl substituiert ist, handelt es sich vor allem um eine 5-Phenyl- und in erster Linie um eine 5-Methyl-2-oxo-1,3-dioxolen-4-ylmethoxy-Gruppe.

Niederalkyl ist z.B. n-Propyl, Isopropyl, n-Butyl, Isobutyl, sek.-Butyl oder tert.-Butyl, ferner auch n-Pentyl, n-Hexyl oder n-Heptyl, in erster Linie jedoch Methyl oder Aethyl.

Niederalkenyl hat 2 - 4 Kohlenstoffatome und ist z.B. Vinyl, Allyl, 2-Methylallyl, n-Propenyl oder Isopropenyl.

Cycloalkyl enthält vorzugsweise 3 bis 8, in erster Linie 5 oder 6 Ringglieder und ist zum Beispiel Cyclopentyl oder Cyclohexyl, ferner Cyclopropyl sowie Cycloheptyl, während Cycloalkenyl z.B. 5 oder 6 Ringglieder und Cycloalkadienyl z.B. 6 Ringglieder enthält und z.B. 1-, 2- oder 3-Cyclohexenyl, 1- oder 2-Cyclopentenyl oder 1,4-Cyclohexadienyl ist.

Phenylniederalkyl ist z.B. Benzyl, 1-Phenyläthyl oder 2-Phenyläthyl.

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy.

Phenylniederalkoxy ist z.B. Benzyloxy oder 1-Phenyläthoxy.

Niederalkanoyloxy ist z.B. Formyloxy, Acetyloxy oder Propionyloxy.

Halogen ist z.B. Fluor, Chlor, Brom oder Jod.

Niederalkylthio ist z.B. Methylthio oder Aethylthio, ferner auch n-Propylthio, Isopropylthio oder n-Butylthio.

Niederalkoxycarbonyl ist z.B. Methoxycarbonyl oder Aethoxycarbonyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkylenamino weist insbesondere 4 bis 6 Kohlenstoffatome auf und ist z.B. Pyrrolidino oder Piperidino.

Niederalkanoylamino ist z.B. Formylamino, Acetylamino oder Propionylamino.

Gegebenenfalls durch Amino und/oder Carboxy substituiertes Niederalkoxycarbonylamino ist z.B. Methoxycarbonylamino, Aethoxycarbonylamino oder Isopropoxycarbonylamino, ferner 2-Amino-2-carboxy-äthoxycarbonylamino.

Niederalkylen als Substituent des Restes $R_4$ kann geradkettig oder verzweigtkettig sein und verbindet beispielsweise 2 vicinale Kohlenstoff- und/oder Heteroatome eines organischen Restes $R_4$ durch 3 bis 5 Kohlenstoffatome. Solche Reste sind beispielsweise 1,3-Propylen, 1,4-Butylen, 1- oder 2-Methyl-1,3-propylen oder 2,2-Dimethyl-1,3-propylen. Eine gemeinsam von den Resten $R_4$ und $R_5$ gebildete, gegebenenfalls durch Hydroxy oder Oxo substituierte Niederalkylen-Gruppe ist insbesondere 1,3-Propylen, 2-Hydroxy-1,3-propylen oder 1-Oxo-1,3-propylen, worin das Kohlenstoffatom 1 der Propylen-Gruppe dem Rest $-\overset{|}{N}-R_6$ benachbart ist.

Gegebenenfalls niederalkyliertes Azaniederalkylen, Oxaniederalkylen, Dioxaniederalkylen oder Thianiederalkylen ist insbesondere geradkettig, kann aber auch verzweigtkettig sein, und verbindet z.B. vicinale Kohlenstoff- und/oder Heteroatome im organischen Rest $R_4$ durch 3 bis 5 Kohlenstoffatome. Solche Reste sind beispielsweise 1- oder 2-Aza-1,3-propylen, 1-Aza-1,4-butylen, 1-Methyl-1-aza, 1-Aethyl-1-aza-, 2-Methyl-2-aza- oder 2-Aethyl-2-aza-1,3-propylen oder 1-Methyl-1-aza- oder 2-Methyl-2-aza-1,4-butylen, ferner 1-Oxa-, 1,3-Dioxa-, 1-Thia- oder 1,3-Dithia-1,3-propylen, oder 1-Oxa-, 1,4-Dioxa-, 1-Thia- oder 1,4-Dithia-1,4-butylen.

N-mono-niederalkyliertes Carbamoyl ist z.B. N-Methyl-, N-Aethyl-oder N-Propylcarbamoyl, während N,N-Di-niederalkyliertes Carbamoyl z.B. N,N-Dimethyl- oder N,N-Diäthylcarbamoyl bedeutet.

Niederalkanoyl ist z.B. Formyl, Acetyl oder Propionyl; Niederalkenoyl ist z.B. Acryloyl; Cycloalkanoyl weist 5-7 Kohlenstoffatome auf und ist z.B. Cyclohexanoyl, während Phenylniederalkanoyl z.B. Phenylacetyl ist.

Niederalkoxysulfonyl ist z.B. Methoxysulfonyl oder Aethoxysulfonyl.

Gegebenenfalls durch Amino und Carboxy substituiertes Niederalkoxycarbonyl als Rest $R_6$ ist z.B. Methoxycarbonyl, Aethoxycarbonyl, n-Propoxycarbonyl oder 2-Amino-2-carboxyäthoxycarbonyl.

Niederalkyl-thiocarbamoyl ist z.B. Methyl-thiocarbamoyl oder Aethylthiocarbamoyl.

Niederalkansulfonyl ist z.B. Methansulfonyl, Aethansulfonyl oder Propansulfonyl.

Geeignete niederaliphatische $R_4$ sind z.B. Niederalkyl, z.B. Methyl, Aethyl, Isopropyl, Isobutyl oder sek.-Butyl, oder durch Hydroxy, Niederalkoxy, z.B. Methoxy oder Aethoxy, Niederalkanoyloxy, z.B. Acetyloxy, Halogen, z.B. Chlor oder Brom, Mercapto, Niederalkylthio, z.B. Methylthio, Amino, Niederalkylamino, z.B. Methyl- oder Aethylamino, Diniederalkylamino, z.B. Dimethylamino, Carbamoylamino, Guanidino, Niederalkanoylamino, z.B. Acetylamino, durch Amino und Carboxy substituiertes Niederalkoxy-, wie Aethoxycarbonylamino, z.B. 2-Amino-2-carboxyäthoxycarbonylamino, Carboxyl, Niederalkoxycarbonyl, z.B. Methoxy- oder Aethoxycarbonyl, Carbamoyl, Sulfo, Sulfamoyl und/oder Oxo substituiertes Niederalkyl, wie insbesondere Methyl, Aethyl, n-Propyl oder n-Butyl. Repräsentative Vertreter solcher niederaliphatischer Reste $R_4$ sind z.B. Methyl, Isopropyl, Isobutyl, sek.-Butyl, Hydroxymethyl, 1-Hydroxyäthyl, Mercaptomethyl, 2-Methylthioäthyl, 3-Aminopropyl, 4-Aminobutyl, 3-Guanidylpropyl, 3-Carbamoylaminopropyl, Carboxymethyl, Carbamoylmethyl, 2-Carboxyäthyl, 2-Carbamoyläthyl, Carboxyl oder Niederalkoxycarbonyl, wie Methoxycarbonyl.

Aromatisch-niederaliphatische Reste $R_4$ sind beispielsweise gegebenenfalls substituiertes Phenylniederalkyl, wie insbesondere -methyl oder -äthyl, worin die Substituenten z.B. Niederalkyl, z.B. Methyl, Nitro, Hydroxy, Niederalkoxy, z.B. Methoxy oder Aethoxy, Carboxy und/oder Halogen, z.B. Chlor, sind.

Geeignete cycloaliphatische Reste $R_4$ sind z.B. Cycloalkenyl, z.B. 1- oder 2-Cyclohexenyl, oder insbesondere Cyclohexadienyl, z.B. 1,4-Cyclohexadienyl.

Aromatische Kohlenwasserstoffreste $R_4$ sind z.B. Phenyl oder durch Amino, Niederalkylamino, z.B. Methyl- oder Aethylamino, Diniederalkylamino, z.B. Dimethylamino, Niederalkyl, z.B. Methyl, Aminoniederalkyl, z.B. 2-Aminoäthyl, Niederalkylaminoniederalkyl, z.B. 2-Methylaminoäthyl, durch Amino und Carboxy substituiertes Niederalkoxycarbonylaminoniederalkyl, z.B. 2-(2-Amino-2-carboxyäthoxycarbonylamino)-äthyl, durch Amino und Carboxy substituiertes Niederalkoxycarbonylamino, z.B. 2-Amino-2-carboxyäthoxycarbonylamino, Nitro, Hydroxy, Niederalkoxy, z.B. Methoxy oder Aethoxy, Halogen, z.B. Chlor oder Brom, oder Carboxy substituiertes, wie insbesondere mono-, aber auch disubstituiertes Phenyl.

Heterocyclische Reste $R_4$ sind insbesondere gegebenenfalls z.B. durch Niederalkyl substituiertes Pyrrolyl oder Dihydropyrrolyl, z.B. 1-Methyl-2-pyrrolyl oder 4,5-Dihydro-3-pyrrolyl, gegebenenfalls z.B. durch Carboxy, Niederalkyl, Aminoniederalkyl oder Amino substituiertes Diazolyl, wie Imidazolyl, z.B. 2-, 4- oder 5-Imidazolyl,

oder 2-Amino-4-imidazolyl, oder Pyrazolyl, z.B. 3-Pyrazolyl, gegebenenfalls z.B. durch Niederalkyl, Amino, Carboxyniederalkyl und/oder Phenyl substituiertes Triazolyl, wie 1H-1,2,3-Triazol-4-yl, 1H-1,2,4-Triazol-3-yl oder 1H-1,2,4-Triazol-5-yl, z.B. die entsprechenden unsubstituierten Reste, 1-Methyl-1H-1,2,3-triazol-4-yl, 5-Methyl- oder 5-Amino-1H-1,2,4-triazol-3-yl, 3-Methyl-1-phenyl-1H-1,2,4-triazol-5-yl, oder 5-Carboxymethyl- oder 5-Phenyl-1H-1,2,4-triazol-3-yl, gegebenenfalls z.B. durch Niederalkyl, Carboxyniederalkyl, Sulfoniederalkyl, Diniederalkylaminoniederalkyl oder gegebenenfalls Substituenten, wie Halogen, enthaltendes Phenyl substituiertes Tetrazolyl, wie 1H- oder 2H-Tetrazol-5-yl, z.B. 1H-Tetrazol-5-yl, 1-Carboxymethyl-, 1-(2-Carboxyäthyl)-, 1-(2-Sulfoäthyl)-, 1-(2-Dimethylaminoäthyl)- oder 1-Phenyl-1H-tetrazol-5-yl, gegebenenfalls z.B. durch Niederalkyl oder Amino substituiertes Thiazolyl, wie 2- oder 4-Thiazolyl, oder Isothiazolyl, wie 3-Isothiazolyl, z.B. 2-Thiazolyl, 2-Amino-4-thiazolyl, 4,5-Dimethyl-2-thiazolyl oder 3-Isothiazolyl, gegebenenfalls z.B. durch Niederalkyl, Hydroxy, Amino oder Niederalkylamino substituiertes Thiadiazolyl, wie 1,2,5-Thiadiazol-3-yl, 1,3,4-Thiadiazol-2-yl, 1,2,4-Thiadiazol-3-yl oder 1,2,4-Thiadiazol-5-yl, z.B. die entsprechenden unsubstituierten Gruppen, ferner 5-Methyl- oder 5-Amino-1,3,4-thiadiazol-2-yl, 3-Methyl-1,2,4-thiadiazol-5-yl, 5-Amino- oder 5-Methylamino-1,2,4-thiadiazol-3-yl oder 4-Hydroxy-1,2,5-thiadiazol-3-yl, gegebenenfalls z.B. durch Amino, Niederalkyl und/oder Phenyl substituiertes Oxazolyl oder Isoxazolyl, wie 2- oder 4-Oxazolyl oder 3-Isoxazolyl, z.B. die entsprechenden unsubstituierten Gruppen, ferner 4-Methyl-2-oxazolyl, 4,5-Diphenyl-2-oxazolyl, 2-Amino-4-oxazolyl oder 5-Methyl-3-isoxazolyl, gegegenenfalls z.B. durch Niederalkyl, Amino oder gegebenenfalls z.B. durch Nitro substituiertes Phenyl substituiertes Oxadiazolyl, wie 1,2,4-Oxadiazol-5-yl, 1,2,4-Oxadiazol-3-yl oder 1,3,4-Oxadiazol-2-yl, z.B. die entsprechenden unsubstituierten Gruppen, ferner 5-Methyl-1,3,4-oxadiazol-2-yl, 5-Phenyl-1,3,4-oxadiazol-2-yl oder 5-(4-Nitrophenyl)-1,3,4-oxadiazol-2-yl, gegebenenfalls z.B. durch Halogen, Niederalkyl oder Aminoniederalkyl substituiertes Furyl oder Thienyl, wie 2- oder 3-Furyl oder 2- oder 3-Thienyl, z.B. die entsprechenden

unsubstituierten Gruppen, 5-Methyl- oder 5-Aminomethyl-2-furyl, oder 5-Aminomethyl-2-thienyl, gegebenenfalls z.B. durch Hydroxy, Halogen, Niederalkyl, Amino und/oder Oxido substituiertes Pyridyl, wie 2-, 3- oder 4-Pyridyl, z.B. die entsprechenden unsubstituierten Gruppen, 1-Oxido-2-pyridyl oder 4-Chlor-1-oxido-2-pyridyl, oder gegebenenfalls z.B. durch Niederalkyl, Amino, Diniederalkylamino, Oxo und/oder Carboxy substituiertes 1,2-Dihydropyrimidyl, wie 1,2-Dihydro-4-pyrimidyl, z.B. 2-Oxo-1,2-dihydro-4-pyrimidyl, 6-Methyl-, 5-Methyl-, 6-Amino-, 6-Dimethylamino-, 5-Carboxy- oder 6-Carboxy-2-oxo-1,2-dihydro-4-pyrimidyl.

Heterocyclisch-niederaliphatische Reste $R_4$ sind z.B. gegebenenfalls substituierte Heterocyclylmethyl-Reste, wie z.B. gegebenenfalls z.B. durch Niederalkyl oder Halogen substituiertes Pyrrol- oder Dihydropyrrol-1-ylmethyl, z.B. Pyrrol-, 3-Methyl-pyrrol-, 3,4-Dichlor-pyrrol-, ferner 2,3- oder 2,5-Dihydropyrrol-1-ylmethyl, gegebenenfalls z.B. durch Niederalkyl substituiertes Diazolylmethyl, wie Imidazol-1-ylmethyl, Imidazol-4-ylmethyl oder Pyrazol-1-ylmethyl, gegebenenfalls z.B. durch Niederalkyl, Carboxyniederalkyl, Amino und/oder Phenyl substituiertes Triazolylmethyl, wie 1H-1,2,3-Triazol-1-ylmethyl, 1H-1,2,4-Triazol-1-ylmethyl oder 1H-1,2,4-Triazol-3-ylmethyl, z.B. die entsprechenden unsubstituierten Reste, 4- oder 5-Methyl-1,2,3-triazol-1-ylmethyl, 5-Methyl-, 5-Amino oder 5-Phenyl-1H-1,2,4-triazol-3-ylmethyl, gegebenenfalls z.B. durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolylmethyl, wie 1H-Tetrazol-1-ylmethyl, 2H-Tetrazol-2-ylmethyl oder 1H-Tetrazol-5-ylmethyl, z.B. die entsprechenden unsubstituierten Reste, 5-Amino-, 5-Carboxymethyl-, 5-(2-Carboxyäthyl)-, 5-Sulfomethyl-, 5-(2-Dimethylaminoäthyl)- oder 5-Phenyl-1H-tetrazol-1-ylmethyl, 5-Amino-, 5-Carboxymethyl-, 5-Sulfomethyl-, 5-(2-Dimethylaminoäthyl)- oder 5-Phenyl-2H-tetrazol-2-ylmethyl, oder 1-(2-Carboxyäthyl)- oder 1-(2-Dimethylaminoäthyl)-2H-tetrazol-5-ylmethyl, unsubstituiertes oder insbesondere durch Oxo und gegebenenfalls zusätzlich z.B. durch Halogen substituiertes Pyridyl oder Dihydropyridylmethyl, wie Pyridiniomethyl, 2-, 3- oder 4-Pyridylmethyl oder 1,2- oder 1,4-Dihydropyrid-1-yl, z.B. 2-Oxo-1,2-dihydropyrid-1-ylmethyl oder 4-Oxo-1,4-dihydropyrid-1-ylmethyl, gegebenenfalls insbesondere durch Oxo und gegebenenfalls zusätzlich z.B. durch Niederalkyl, Amino, Diniederalkylamino oder Carboxy substituiertes Dihydropyrimid-1-ylmethyl, wie 2H-1,2-Dihydro- oder 4H-1,4-Dihydro-pyrimid-1-ylmethyl, z.B. 2-Oxo-1,2-dihydro-pyrimid-1-ylmethyl, 6-Methyl-, 5-Methyl-, 6-Amino-, 6-Dimethylamino-, 5-Carboxy- oder 6-Carboxy-2-oxo-1,2-dihydro-pyrimid-1-ylmethyl oder 4-Oxo-1,4-dihydro-pyrimid-1-ylmethyl, Furylmethyl, z.B. 2-Furylmethyl, Thienylmethyl, z.B. 2-Thienylmethyl, gegebenenfalls durch z.B. Amino substituiertes Oxazolylmethyl, Thiazolylmethyl oder Thiadiazolylmethyl, z.B. 2-Amino-oxazol-4-ylmethyl, 2-Amino-thiazol-4-ylmethyl oder 5-Amino-1,2,4-thiadiazol-3-ylmethyl, oder Indolylmethyl, z.B. Indol-3-ylmethyl.

Bevorzugt als Reste $R_1$ sind Hydroxymethyl und in erster Linie 1-Hydroxyäthyl.

Bevorzugte unter physiologischen Bedingungen spaltbare veresterte Carboxylgruppen $R_2$ sind z.B. Niederalkanoyloxymethoxycarbonyl, z.B. Acetoxymethoxycarbonyl oder Pivaloyloxymethoxycarbonyl, und 1-Niederalkoxycarbonyloxyniederalkoxycarbonyl, z.B. 1-Aethoxycarbonyloxyäthoxycarbonyl.

Bevorzugt als Rest $R_3$ ist Wasserstoff.

In bevorzugten Verbindungen der Formel (I) steht der Rest $-C(=O)-CH(-R_4)-N(R_5,R_6)$ für den Acylrest einer natürlichen, insbesondere einer genetisch enkodierten α-Aminosäure, wie z.B. für den Acylrest von Glycin, Alanin, Valin, Prolin, Serin, Threonin, Methionin, Tryptophan, Tyrosin, Asparagin, Asparginsäure, Glutamin, Glutaminsäure, Lysin, Arginin oder Histidin, ferner für den Acylrest von Homoserin, Phenylglycin, 4-Hydroxyphenylglycin, α-Aminobuttersäure, Citrullin, Ornithin, 3-Hydroxyprolin oder Pyrrolidin-5-on-2-carbonsäure, sowie N-niederalkylierten Derivaten, wie den

N-Methyl-Derivaten, davon. Solche Acylreste können sowohl in der natürlichen, d.h. L-Konfiguration, als auch in der D-Konfiguration oder als Gemisch beider Konfigurationen (DL-Form) vorliegen.

Die in den Verbindungen der Formel I vorhandenen funktionellen Gruppen, wie Hydroxy-, Carboxy-, Amino- oder Sulfogruppen, insbesondere die Hydroxygruppe im Rest $R_1$ und die Carboxylgruppe $R_2$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe im Rest $R_1$, ferner eine im Rest $R_4$ oder $R_6$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbutyl)-silyl oder tert.-Butyl-dimethylsilyl, 2-Halogenniederalkylgruppen, z.B. 2-Chlor-, 2-Brom-, 2-Jod- und 2,2,2-Trichloräthyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist Triniederalkylsilyl, Niederalkenyloxycarbonyl, Niederalkenyloxyoxalyl und durch Halogen substituiertes Niederalkoxycarbonyl.

Eine Carboxylgruppe $R_2$ oder $R_4$, ferner auch eine im Rest $R_4$ oder $R_6$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist. Eine geschützte Carboxylgruppe kann ferner eine leicht in eine andere funktionell abgewandelte Carboxylgruppe, wie in eine andere veresterte Carboxylgruppe umwandelbare, veresterte Carboxylgruppe darstellen.

Solche veresterten Carboxylgruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls, z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methylsilyl)-äthoxycarbonyl.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylgruppen sind in erster Linie Triniederalkylsilyl, insbesondere Trimethylsilyl oder Dimethyl-tert.-butylsilyl, oder entsprechend substituierte Stannylgruppen, z.B. Tri-n-butylstannyl. Bevorzugte geschützte Carboxylgruppen $R_2^1$ sind die 4-Nitrobenzyloxycarbonyl-, Niederalkenyloxycarbonyl-, insbesondere Allyloxycarbonyl-, und die in 2-Stellung durch Niederalkylsulfonyl, Cyano oder Triniederalkylsilyl, z.B. Trimethylsilyl oder Di-n-butyl-methylsilyl, substituierte Aethoxycarbonylgruppe.

Eine geschützte Aminogruppe kann beispielsweise in Form einer leicht spaltbaren Acylamino-, Acylimino-, verätherten Mercaptoamino-, Silyl- oder Stannylaminogruppe oder als Enamino-, Nitro- oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkysilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -äthylhalbesters, und Niederalk-1-en insbesondere 1-Propen bedeutet. Entsprechende Schutzgruppen sind in erster Linie 1-Niederalkanoylprop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Bevorzugte geschützte Aminogruppen sind z.B. Azido, Phthalimido, Nitro, Niederalkenyloxycarbonylamino, z.B. Allyloxycarbonylamino, und gegebenenfalls durch Nitro substituiertes Benzyloxycarbonylamino.

Eine geschützte Sulfogruppe im Rest $R_4$ oder $R_6$ ist in erster Linie eine veresterte, wie mit einem aliphatischen, cycloaliphatischen, cycloaliphatisch-aliphatischen, aromatischen oder araliphatischen Alkohol, z.B. einem Niederalkanol, oder mit einem Silyl- oder Stannylrest, wie Triniederalkysilyl, veresterte Sulfogruppe. In einer Sulfogruppe kann die Hydroxygruppe beispielsweise wie die Hydroxygruppe in einer veresterten Carboxylgruppe veräthert sein.

Salze von erfindungsgemässen Verbindungen sind in erster Linie pharmazeutisch annehmbare, nicht-toxische Salze von Verbindungen der Formel I. Solche Salze werden beispielsweise von den in Verbindungen der Formel I vorhandenen sauren Gruppen, z.B. Carboxyl- und Sulfogruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-ß-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können im Substituenten $R_1$ ein zusätzliches Chiralitätszentrum besitzen. Beispielsweise kann 1-Hydroxyäthyl als Substituent $R_1$ in der R-, in der S- oder in der racemischen R,S-Konfiguration vorliegen. In bevorzugten Penem-Verbindungen der Formel I weist ein Rest $R_1$, welcher ein asymmetrisches Kohlenstoffatom besitzt, insbesondere 1-Hydroxyäthyl, die R-Konfiguration auf.

Verbindungen der Formel I, worin $R_4$ von Wasserstoff verschieden ist, besitzen am Methin-Kohlenstoffatom $-CH(R_4)(NR_5R_6)$ ein weiteres Chiralitätszentrum, dass in der R-, in der S- oder in der racemischen R,S-Konfiguration vorliegen kann. Gegebenenfalls in den Resten $R_4$, $R_5$ und/oder $R_6$ enthaltene weitere Chiralitätszentren können ebenfalls entweder in der R-, S- oder in der R,S-Konfiguration vorliegen.

Die Erfindung betrifft demgemäss die reinen Diastereomeren und Mischungen von Diastereomeren von Verbindungen der Formel I, welche im Rest $R_1$ und/oder im Rest $-CH(-R_4)-$ zusätzliche Chiralitätszentren aufweisen.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, $R_3$, $R_4$, $R_5$ und $R_6$ die unter Formel I angegebene Bedeutungen haben, wobei funktionelle Gruppen in ungeschützter Form vorliegen, und $R_2$ Carboxyl oder eine unter physiologischen Bedingungen spaltbare veresterte Carboxylgruppe darstellt, und pharmakologisch annehmbare Salze von solchen Verbindungen mit salzbildenden Gruppen haben antibakterielle Wirkungen.

Beispielsweise sind sie in vitro gegen grampositive und gramnegative Kokken, z.B. Staphylococcus aureus, Streptococcus pyogenes, Streptococcus pneumoniae und Streptococcus faecalis, in minimalen Konzentrationen von ca. 0,05 bis ca. 16 µg/ml und gegen gramnegative Stäbchenbakterien, wie Enterobacteriaceae, Haemophilus influenzae und Pseudomonas aeruginosa, und Anaerobier, z.B. Bacteroides sp., in minimalen Konzentrationen von ca. 0,2 bis ca. 32 µg/ml wirksam. In vivo, bei der systemischen Infektion der Maus, z.B. durch Staphylococcus aureus, ergeben sich bei subkutaner oder oraler Applikation erfindungsgemässer Verbindungen $ED_{50}$-Werte von ca. 1 bis ca. 10 mg/kg.

Ueberraschenderweise weisen die Verbindungen der Formel I, worin $R_1$, $R_2$, $R_3$, $R_4$, $R_5$ und $R_6$ die angegebenen Bedeutungen haben, wobei funktionelle Gruppen in ungeschützter Form vorliegen, nicht nur in der pro-drug-Form ($R_2$ ist unter physiologischen Bedingungen spaltbares verestertes Carboxyl) sondern auch in der freien Form ($R_2$ is Carboxyl) eine ausgezeichnete Resorption bei oraler Applikation auf. Eine nennenswerte Resorption bei oraler Applikation ist im Falle der Peneme bisher im wesentlichen nur bei der aus dem US-Patent Nr. 4 374 844 bekannten (5R,6S)-2-Aethylthio-6-[(1R)-1-hydroxyäthyl]-2-penem-3-carbonsäure (Resorptionsrate ca. 9 %) und bei pro-drug-Formen von Penemen bekannt geworden. So beträgt die Resorption der nicht-acylierten Ausgangspeneme (5R,6S)-2-Aminomethyl-6-hydroxymethyl-2-penem-3-carbonsäure bzw. (5R,6S)-2-Aminomethyl-6-[(1R)-1-hydroxyäthyl]-2-penem-3-carbonsäure (bekannt aus der Deutschen Offenlegungsschrift Nr. 3431980) nach oraler Applikation in der Maus nur ca. 2,5 %. Demgegenüber werden die Peneme der vorliegenden Erfindung, in denen $R_2$ Carboxyl ist, nach oraler Applikation in der Maus zu etwa 15 - 40 % resorbiert.

Die neuen Verbindungen können daher als parenteral und insbesondere auch als oral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die Erfindung betrifft vor allem Verbindungen der Formel I, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl $R_2$ bedeutet; $R_3$ Wasserstoff oder Niederalkyl ist; $R_4$ Wasserstoff oder Niederalkyl, Niederalkenyl, Cycloalkyl, Cycloalkenyl, Cycloalkadienyl, Phenyl, Naphthyl, Phenylniederalkyl, einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom, ausgewählt aus der Gruppe Sauerstoff und Schwefel, z.B. einen entsprechenden aza-, diaza-, triaza-, tetraza-, oxa-, oxaza-, oxadiaza-, oxatriaza-, thia-, thiaza-, thiadiaza- oder thiatriazacyclischen Rest aromatischen Charakters oder einen entsprechenden Dihydro- oder Tetrahydrorest, oder ein gegebenenfalls partiell gesättigtes Benzo-, Dibenzo-, Pyrido- oder Pyrimido-Derivat eines solchen 5- oder 6-gliedrigen Restes, oder einem durch einen der genannten, über ein Ringkohlenstoffatom gebundenen, gegebenenfalls partiell gesättigten Heteroarylreste oder durch einen über ein Ringstickstoffatom gebundenen monocyclischen, 5- oder 6-gliedrigen gegebenenfalls partiell gesättigten Heterocyclylrest mit 1 - 4 Ringstickoffatomen substituierter Niederalkylrest darstellt, welche Reste $R_4$ unsubstituiert oder durch Hydroxy, Niederalkoxy, Phenylniederalkoxy, Niederalkanoyloxy, Halogen, Mercapto, Niederalkylthio, Phenylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl, Niederalkylen, gegebenenfalls niederalkyliertes Azaniederalkylen, Oxaniederalkylen, Dioxaniederalkylen, Thianiederalkylen, Amino,

Niederalkylamino, Diniederalkylamino, Niederalkylenamino, Carbamoylamino, Guanidino, Niederalkanoylamino, gegebenenfalls durch Amino und/oder Carboxy substituiertes Niederalkoxycarbonylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Cycloalkyl, Nitro, Oxo und/oder Oxido substituiert sind; $R_5$ Wasserstoff, Niederalkyl oder zusammen mit $R_4$ gegebenenfalls durch Hydroxy oder Oxo substituiertes Niederalkylen ist; und $R_6$ Wasserstoff, Niederalkyl, Niederalkanoyl, durch Hydroxy, Niederalkoxy, Amino, Halogen, Carboxy und/oder Cyan substituiertes Niederalkanoyl; Niederalkenoyl, Cycloalkanoyl, Benzoyl, durch Amino, Niederalkylamino, Diniederalkylamino, Niederalkanoylamino, Carboxy, Halogen, Nitro, Cyano und/oder Niederalkyl substituiertes Benzoyl, gegebenenfalls durch Amino oder Hydroxy substituiertes Phenylniederalkanoyl, Heterocyclylcarbonyl, worin Heterocyclyl einer der bei der Definition von $R_4$ angegebenen gegebenenfalls partiell gesättigten, gegebenenfalls substituierten 5- oder 6-gliedrigen monocyclischen Heteroarylreste oder ein gegebenenfalls durch Oxo, Sulfo, Niederalkoxysulfonyl, Sulfamoyl, Niederalkyl und/oder Hydroxy substituierter gesättigter 5- oder 6-gliedriger Diazacycloalkan-Rest bedeutet; Niederalkoxycarbonyl, durch Carboxy und Amino substituiertes Niederalkoxycarbonyl, Benzyloxycarbonyl, Carbamoyl, Anilinocarbonyl, N-mono- oder N,N-diniederalkyliertes Carbamoyl, Niederalkyl-thiocarbamoyl, Sulfo, Niederalkansulfonyl, durch Halogen substituiertes Niederalkansulfonyl, Benzolsulfonyl, durch Amino, Halogen, Niederalkyl oder Nitro substituiertes Benzolsulfonyl, Sulfamoyl oder eine Aminoschutzgruppe ist, optische Isomere von Verbindungen der Formel I, welche insbesondere im Rest $R_1$ und/oder am Methin-Kohlenstoffatom $-CH(R_4)(NR_5R_6)$ Chiralitätszentren besitzen, Mischungen dieser optischen Isomere und Salze von solchen Verbindungen der Formel I, die eine salzbildende Gruppe aufweisen.

Die Erfindung betrifft hauptsächlich Verbindungen der Formel I, worin $R_1$ in α-Stellung durch Hydroxy oder geschütztes Hydroxy, z.B. Triniederalkylsilyloxy, 2-Halogenniederalkoxycarbonyloxy, Niederalkenyloxycarbonyloxy oder gegebenenfalls durch Nitro substituiertes Phenylniederalkoxycarbonyloxy, substituiertes Niederalkyl ist; $R_2$ Carboxyl, geschütztes Carboxyl, z.B. Niederalkenyloxycarbonyl, gegebenenfalls durch Nitro substituiertes Benzyloxycarbonyl, Niederalkanoylmethoxycarbonyl, 2-Halogenniederalkoxycarbonyl oder 2-Triniederalkylsilyläthoxycarbonyl, oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet; $R_3$ Wasserstoff oder Niederalkyl ist; $R_4$ Wasserstoff, Niederalkyl, durch Hydroxy, Niederalkoxy, Halogen, Mercapto, Niederalkylthio, Amino, Niederalkylamino, Diniederalkylamino, Niederalkanoylamino, Guanidino, Carbamoylamino, Carboxy, Niederalkoxycarbonyl, Carbamoyl, Sulfo und/oder Oxo substituiertes Niederalkyl, Cycloalkenyl mit 5 oder 6 Kohlenstoffatomen, Cycloalkadienyl mit 6 Kohlenstoffatomen, Phenyl, durch Hydroxy, Niederalkoxy, Amino, Niederalkylamino, Diniederalkylamino, Aminoniederalkyl, durch Amino und Carboxy substituiertes Niederalkoxycarbonylamino oder durch Carboxy und Amino substituiertes Niederalkoxycarbonylaminoniederalkyl substituiertes Phenyl; gegebenenfalls durch Niederalkyl, Nitro, Amino, Hydroxy, Niederalkoxy, Carboxy und/oder Halogen substituiertes Phenylniederalkyl; über ein Ringkohlenstoffatom gebundenes, gegebenenfalls durch Niederalkyl, Aminoniederalkyl oder Amino substituiertes Imidazolyl, z.B. 2- oder 4-Imidazolyl, Pyrazolyl, z.B. 3-Pyrazolyl, gegebenenfalls durch Amino substituiertes Triazolyl, wie 1H-1,2,4-Triazol-3-yl oder 1H-1,2,4-Triazol-5-yl, gegebenenfalls durch Niederalkyl, Carboxyniederalkyl, Sulfoniederalkyl oder Diniederalkylaminoniederalkyl substituiertes Tetrazolyl, wie 1H- oder 2H-Tetrazol-5-yl, gegebenenfalls durch Niederalkyl oder Amino substituiertes Thiazolyl, wie 2- oder 4-Thiazolyl, Isothiazolyl, wie 3-Isothiazolyl, gegebenenfalls durch Niederalkyl, Hydroxy, Amino oder Niederalkylamino substituiertes Thiadiazolyl, wie 1,2,5-Thiadiazol-3-yl, 1,3,4-Thiadiazol-2-yl oder 1,2,4-Thiadiazol-3-yl, gegebenenfalls durch Amino oder Niederalkyl substituiertes Oxazolyl, wie 2- oder 4-Oxazolyl, gegebenenfalls durch Niederalkyl oder Amino substituiertes Oxadiazolyl, wie 1,2,4-Oxadiazol-5-yl oder 1,2,4-Oxadiazol-3-yl, gegebenenfalls durch Halogen, Niederalkyl oder Aminoniederalkyl substituiertes Furyl, wie 2- oder 3-Furyl, gegebenenfalls durch Halogen, Niederalkyl oder Aminoniederalkyl substituiertes Thienyl, wie 2- oder 3-Thienyl, gegebenenfalls durch Halogen, Amino und/oder Oxido substituiertes Pyridyl, wie 2-, 3-oder 4-Pyridyl; gegebenenfalls durch Niederalkyl substituiertes Imidazol-4-ylmethyl, gegebenenfalls durch Oxo und gegebenenfalls zusätzlich durch Halogen substituiertes Pyridyl- oder Dihydropyridylmethyl, wie Pyridinio-, Pyrid-2-yl-, Pyrid-3-yl-, Pyrid-4-yl-oder Dihydropyrid-1-ylmethyl, Furylmethyl, z.B. 2-Furylmethyl, Thienylmethyl, z.B. 2-Thienylmethyl, gegebenenfalls durch Amino substituiertes Oxazolyl-, Thiazolyl- oder Thiadiazolylmethyl, z.B. Oxazolyl-4-, Thiazolyl-4-oder 1,2,4-Thiadiazol-3-ylmethyl, oder Indol-3-ylmethyl darstellt; $R_5$ Wasserstoff, Niederalkyl oder zusammen mit $R_4$ gegebenenfalls durch Hydroxy oder Oxo substituiertes Niederalkylen ist; $R_6$ Wasserstoff, Niederalkyl, Niederalkanoyl, durch Hydroxy, Carboxy und/oder Amino substituiertes Niederalkanoyl, Phenylniederalkanoyl, worin Niederalkanoyl in α-Stellung zum Phenylrest durch Hydroxy oder Amino substituiert ist, gegebenenfalls durch Hydroxy und/oder Niederalkyl substituiertes Pyridylcarbonyl, z.B. Nicotinoyl oder Isonicotinoyl, Furoyl, z.B. 2-Furoyl, Thenoyl, z.B. 2-Thenoyl, durch Carboxy substituiertes Imidazolylcarbonyl, z.B. Imidazol-5-ylcarbonyl, durch Oxo, Sulfamoyl und/oder Niederalkyl substituiertes Tetrahydroimidazolylcarbonyl, z.B. Tetrahydroimidazol-1-ylcarbonyl, durch Hydroxy und/oder Niederalkyl substituiertes Piperazinylcarbonyl, z.B. Piperazin-1-ylcarbonyl, durch Hydroxy substituiertes Thiadiazolylcarbonyl, z.B. 1,2,5-Thiadiazol-3-ylcarbonyl, Niederalkoxycarbonyl, durch Amino und Carboxy substituiertes Niederalkoxycarbonyl, Benzyloxycarbonyl, Carbamoyl, Niederalkylcarbamoyl, Niederalkansulfonyl oder Sulfamoyl ist; optische Isomere von Verbindungen der Formel I, welche insbesondere im Rest $R_1$ und/oder am Methin-Kohlen-

stoffatom $-CH(R_4)(NR_5R_6)$ Chiralitätszentren besitzen, Mischungen dieser optischen Isomere und Salze von solchen Verbindungen der Formel I, die eine salzbildende Gruppe aufweisen.

Die Erfindung betrifft insbesondere Verbindungen der Formel I, worin $R_1$ Hydroxymethyl oder 1-Hydroxyäthyl ist, $R_2$ Carboxyl oder unter physiologischen Bedingungen spaltbares verestertes Carboxyl, z.B. 1-Niederalkoxycarbonyloxyniederalkoxycarbonyl oder Niederalkanoyloxymethoxycarbonyl, ist; $R_3$ Wasserstoff bedeutet; $R_4$ Wasserstoff, Niederalkyl, z.B. Methyl oder Isopropyl, durch Hydroxy, Mercapto, Niederalkylthio, Amino, Guanidino, Carbamoylamino, Carboxy oder Carbamoyl substituiertes Niederalkyl, z.B. Hydroxy-, Mercapto-, Carboxy- oder Carbamoylmethyl, 1-Hydroxy-, 2-Methylthio-, 2-Carboxy- oder 2-Carbamoyläthyl, 4-Aminobutyl oder 3-Guanidinopropyl; Cyclohexadienyl, gegebenenfalls durch Hydroxy oder Amino substituiertes Phenyl, gegebenenfalls durch Hydroxy substituiertes Phenylmethyl, über ein Kohlenstoffatom gebundenes Aminooxazolyl, z.B. 2-Amino-4-oxazolyl, Aminothiazolyl, z.B. 2-Amino-4-thiazolyl, Aminothiadiazolyl, z.B. 5-Amino-1,2,4-thiadiazol-3-yl, Hydroxythiadiazolyl, z.B. 4-Hydroxy-1,2,5-thiadiazol-3-yl, Pyridyl, z.B. 3- oder 4-Pyridyl, Aminoimidazolyl, z.B. 2-Amino-imidazol-4-yl, Imidazol-4-ylmethyl, Aminothiazol-4-ylmethyl, z.B. 2-Amino-thiazol-4-ylmethyl, oder Indol-3-ylmethyl bedeutet; $R_5$ Wasserstoff oder zusammen mit $R_4$ 1,3-Propylen oder 2-Hydroxy-1,3-propylen bedeutet; und $R_6$ Wasserstoff, Methyl oder Niederalkanoyl, z.B. Acetyl, ist, optische Isomere von Verbindungen der Formel I, worin $R_1$ 1-Hydroxyäthyl ist und/oder welche am Methin-Kohlenstoffatom $-CH(R_4)(NR_5R_6)$ ein Chiralitätszentrum besitzen, Mischungen dieser optischen Isomere und Salze von solchen Verbindungen der Formel I, die eine salzbildende Gruppe aufweisen.

Die Erfindung betrifft hauptsächlich Verbindungen der Formel (I), worin $R_1$ Hydroxymethyl oder 1-Hydroxyäthyl bedeutet, $R_2$ Carboxyl oder eine unter physiologischen Bedingungen spaltbare veresterte Carboxylgruppe ist und der Rest $-C(=O)-(CH-R_4)-N(R_5,R_6)$ für den Acylrest einer genetisch enkodierten Aminosäure, für α-Aminobutyryl,

Phenylglycyl, 4-Hydroxyphenylglycyl, Citrullyl, Ornithyl, 3-Hydroxyprolyl, Pyrrolidin-5-on-2-carbonyl oder für ein N-Niederalkyl-Derivat eines solchen Acylrestes steht, optische Isomere von Verbindungen der Formel (I), worin $R_1$ 1-Hydroxyäthyl ist und/oder welche am Methin-Kohlenstoffatom $-CH(R_4)(NR_5R_6)$ ein Chiralitätszentrum besitzen, Mischungen solcher optischen Isomere, und Salze von solchen Verbindungen der Formel (I), die eine salzbildende Gruppe aufweisen.

Die Erfindung betrifft in erster Linie Verbindungen der Formel I, worin $R_1$ Hydroxymethyl oder 1-Hydroxyäthyl ist, $R_2$ Carboxyl bedeutet, $R_3$, $R_5$ und $R_6$ Wasserstoff sind und $R_4$ Wasserstoff, Methyl, Hydroxymethyl oder Imidazol-4-ylmethyl darstellt, optische Isomere von Verbindungen der Formel I, worin $R_1$ 1-Hydroxyäthyl bedeutet und/oder worin $R_4$ von Wasserstoff verschieden ist, Mischungen dieser optischen Isomere und Salze von solchen Verbindungen der Formel I, die eine salzbildende Gruppe aufweisen.

Die Erfindung betrifft vor allem die in den Beispielen genannten Verbindungen der Formel I und ihre pharmazeutisch annehmbaren Salze.

Die Verbindungen der vorliegenden Erfindung können nach an sich bekannten Verfahren hergestellt werden.

Die neuen Verbindungen werden z.B. hergestellt, indem man

a) in einer Verbindung der Formel

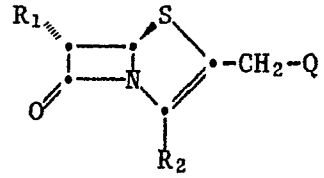

(II),

worin $R_1$ und $R_2$ die unter Formel (I) angegebene Bedeutung haben und Q eine in den Rest

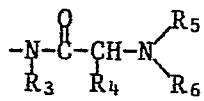 (III)

überführbare Gruppe ist, die Gruppe Q in den Rest

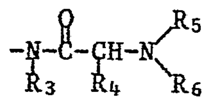 (III)

überführt, oder

b) eine Ylid-Verbindung der Formel

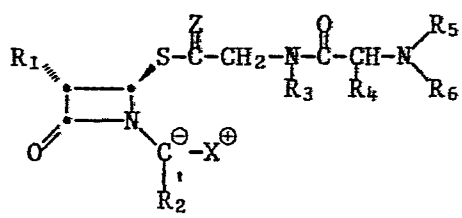 (V) ,

worin $R_1$, $R_3$, $R_4$, $R_5$ und $R_6$ die unter Formel I angegebenen Bedeutungen haben, $R_2$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

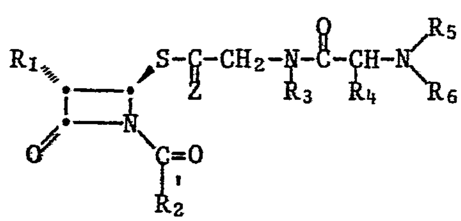 (VI) ,

worin $R_1$, $R_3$, $R_4$, $R_5$ und $R_6$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, oder

d) in einer Verbindung der Formel

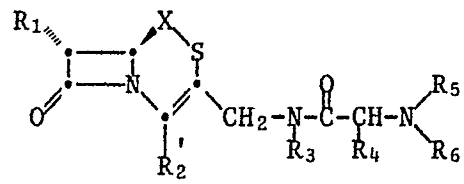

(VII) ,

worin $R_1$, $R_3$, $R_4$, $R_5$ und $R_6$ die unter Formel I angegebenen Bedeutungen haben, $R_2$ eine geschützte Carboxylgruppe darstellt und X für die Gruppe -S- oder für die Gruppe $-SO_2-$ steht, den Rest X abspaltet, und wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/ oder, wenn erwünscht, in einer erhältlichen Verbindung der Formel I einen Rest $R_3$ und/oder $R_4$ und/oder $R_5$ und/oder $R_6$ in einen anderen Rest $R_3$ und/oder $R_4$ und/oder $R_5$ und/oder $R_6$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

In den Ausgangsverbindungen der Formeln II, V - VII sind funktionelle Gruppen, wie freie Hydroxygruppen, z.B. im Rest $R_1$, und freie Aminogruppen vorzugsweise durch konventionelle Schutzgruppen, z.B. durch eine der oben genannten, geschützt.

a) <u>Aufbau der Seitenkette</u>

Eine in einen Rest

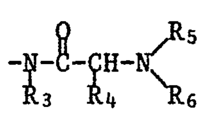

(III)

überführbare Gruppe Q ist insbesondere eine Gruppe $-NHR_3$ oder eine entsprechende, durch nucleophile Reaktion austauschbare Gruppe. Solche austauschbaren Gruppen Q sind insbesondere veresterte Hydroxygruppen, wie durch eine Halogenwasserstoffsäure, eine Diniederalkyl- oder Diarylphosphorsäure, eine gegebenenfalls durch Oxo oder Halogen substituierte Niederalkancarbonsäure, eine gegebenenfalls durch Halogen substituierte Niederalkan- oder eine gegebenenfalls durch Halogen oder Niederalkyl substituierte Benzolsulfonsäure veresterte Hydroxygruppen. Solche Gruppen Q sind z.B. Halogen, wie Chlor, Brom oder Jod, Diniederalkyl- oder Diarylphosphoryloxy, z.B. Dimethyl-, Diäthyl- oder Diphenylphosphoryloxy, gegebenenfalls durch Halogen oder Oxo substituiertes Niederalkanoyloxy, z.B. Formyloxy, Acetyloxy oder Acetacetyloxy, gegebenenfalls durch Halogen substituiertes Niederalkansulfonyloxy, z.B. Methansulfonyloxy oder Trifluormethansulfonyloxy, oder gegebenenfalls durch Halogen oder Niederalkyl substituiertes Benzolsulfonyloxy, z.B. Benzolsulfonyloxy, 4-Chlor-, 4-Brom- oder 4-Methylbenzolsulfonyloxy.

Die Umsetzung einer Verbindung der Formel II, worin Q eine durch nucleophile Reaktion austauschbare Gruppe ist, erfolgt z.B. mit einer Verbindung der Formel

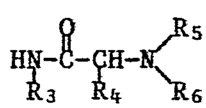

(VIII) ,

worin funktionelle Gruppen in den Resten $R_4$, $R_5$ und $R_6$ in geschützter Form vorliegen, in Gegenwart eines basischen Kondensationsmittels, beispielsweise eines Alkalimetall-niederalkanolats, z.B. Natrim-methanolat oder Kalium-tert.butanolat, oder eines Alkalimetallhydrids oder -amids, z.B Natriumhydrid oder Natriumamid, in einem inerten Lösungsmittel, z.B. einem Aether, wie Dioxan oder Tetrahydrofuran, einem Kohlenwasserstoff, wie Benzol, oder einem Amid, z.B. Formamid oder Dimethylformamid, bei Verwendung von Alkalimetall-niederalkanolats auch im entsprechenden Niederalkanol, oder Lösungsmittelgemisch bei Raumtemperatur oder erniedrigter oder

leicht erhöhter Temperatur, z.B. bei etwa -60°C bis etwa +40°C, insbesondere bei etwa -20° bis etwa +30°C, falls erforderlich in einer Inertgas-, wie Stickstoffatmosphäre.

Man kann auch in einer Verbindung der Formel II, worin Q die Gruppe $-NHR_3$ ist, diese Aminogruppe durch Umsetzen mit einem den Acylrest einer Carbonsäure der Formel

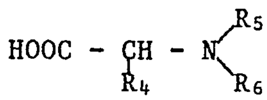

(IV)

einführenden Acylierungsmittel acylieren. Ein den Acylrest einer Carbonsäure der Formel IV einführendes Acylierungsmittel ist entweder die Carbonsäure der Formel IV selbst oder eine reaktionsfähiges funktionelles Derivat oder ein Salz davon.

Falls eine freie Säure der Formel IV zur Acylierung eingesetzt wird, wird die Reaktion üblicherweise in Gegenwart von geeigneten Kondensationsmitteln, wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, durchgeführt.

Die Kondensationsreaktion wird vorzugsweise in einem wasserfreien Reaktionsmedium, vorzugsweise in einem inerten Lösungsmittel, z.B. Methylenchlorid, Dimethylformamid, Acetonitrol oder Tetrahydrofuran, wenn erwünscht oder notwendig, unter Kühlen oder Erwärmen, z.B. in einem Temperaturbereich von etwa -40°C bis etwa +100°C, bevorzugt von etwa -20°C bis etwa +50°C, und, falls erforderlich, in einer Inertgas-, z.B. Stickstoffatmosphäre, durchgeführt.

Ein reaktionsfähiges, d.h. ein Carboxamid-bildendes, funktionelles Derivat einer Carbonsäure der Formel IV ist in erster Linie ein Anhydrid der Carbonsäure der Formel IV, z.B. ein gemischtes Anhydrid, ferner ein N-Carbonsäure-Anhydrid. Ein gemischtes Anhydrid wird beispielsweise durch Kondensation mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder -bromid. Ein gemischtes Anhydrid wird ferner durch Kondensation mit Stickstoffwasserstoffsäure gebildet und ist beispielsweise das Carbonsäureazid. Weitere anorganische Säuren, die sich zur Bildung gemischter Anhydride eignen, sind Phosphor-haltige Säuren, z.B. Phosphorsäure, Diäthylphosphorsäure oder phosphorige Säure, Schwefel-haltige Säuren, z.B. Schwefelsäure, oder Cyanwasserstoffsäure. Ein reaktionsfähiges, funktionelles Derivat einer Carbonsäure der Formel IV wird ausserdem durch Kondensation mit einer organischen Carbonsäure gebildet, z.B. mit einer unsubstituierten oder durch Halogen, z.B. Fluor oder Chlor, oder Cyan substituierten Niederalkancarbonsäure, z.B. Pivalinsäure, Isovaleriansäure, Trifluoressigsäure oder Cyanessigsäure, mit einem Niederalkylhalbester der Kohlensäure, z.B. dem Aethyl-oder Isobutylhalbester der Kohlensäure, oder mit einer organischen, z.B. aliphatischen oder aromatischen Sulfonsäure, z.B. Methansulfonsäure oder p-Toluolsulfonsäure. Ein N-Carbonsäure-Anhydrid einer Säure der Formel IV wird beispielsweise aus dem N-Benzyloxycarbonyl-Derivat der Säure durch Umsetzen mit Dichlormethyl-methyl-äther oder Thionylchlorid erhalten.

Ein reaktionsfähiges funktionelles Derivat einer Carbonsäure der Formel IV ist ebenfalls ein entsprechender aktivierter Ester, der beispielsweise durch Kondensation mit einem vinylogen Alkohol, d.h. mit einem Enol, z.B. einem vinylogen Niederalkenol, gebildet wird, ein Iminomethylesterhalogenid, z.B. Dimethyliminomethylesterchlorid, hergestellt aus der Carbonsäure der Formel IV und z.B. Dimethyl-(1-chloräthyliden)-iminiumchlorid der Formel $[(CH_3)_2N{=}C(Cl)CH_3]Cl$ , das man seinerseits z.B. aus N,N-Dimethyl-

ester, z.B. ein durch Halogen, z.B. Chlor, und/oder Nitro substituierter Phenylester, z.B. ein Pentachlor-, 4-Nitrophenyl- oder 2,4-Dinitrophenylester, ein N-heteroaromatischer Ester, z.B. N-Benztriazolester, oder ein N-Diacyliminoester, z.B. ein N-Succinimino- oder N-Phthaliminoester.

Die Acylierung mit einem reaktionsfähigen funktionellen Derivat der Carbonsäure der Formel IV, z.B. mit einem entsprechenden N-Carbonsäure-Anhydrid oder einem entsprechenden Säurehalogenid, wird bevorzugt in Anwesenheit eines geeigneten säurebindenden Mittels, beispielsweise einer geeigneten organischen Base, durchgeführt. Eine geeignete organische Base ist beispielsweise ein Amin, z.B. ein tertiäres Amin, wie Triniederalkylamin, z.B. Trimethylamin, Triäthylamin oder Aethyl-diisopropylamin, oder ein N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin. Ein geeignetes säurebindendes Mittel ist ferner eine anorganische Base, beispielsweise ein Alkalimetall- oder Erdalkalimetallhydroxid, -carbonat oder -hydrogencarbonat, z.B. Natrium-, Kalium- oder Calciumhydroxid, -carbonat oder -hydrogencarbonat. Bei Verwendung eines N-Carbonsäure-Anhydrides einer Säure der Formel IV arbeitet man in einem alkalischen Puffer bei etwa pH 10. Die Acylierung wird vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, wenn notwendig oder erwünscht bei erniedrigter oder erhöhter Temperatur, z.B. in einem Temperaturbereich von etwa -40° bis etwa +60°C, bevorzugt von etwa -10° bis etwa +30°C, und, falls erforderlich, in einer Inertgas-, z.B. Stickstoffatmosphäre.

Die Decarboxylierung des bei Verwendung eines N-Carbonsäure-Anhydrides erhältlichen Primärproduktes erfolgt bei gleicher Temperatur durch Ansäuren der Reaktionsmischung auf, z.B., pH 5.

Ein bei der Acylierungsreaktion eingesetztes, reaktionsfähiges funktionelles Derivat einer Säure der Formel IV kann, wenn erwünscht, in situ gebildet werden. So kann man z.B. ein gemischtes Anhydrid in situ herstellen, indem man eine Säure der Formel IV, worin funktionelle Gruppen gegebenenfalls geschützt sind, oder ein geeignetes Salz davon, z.B. ein Ammoniumsalz, welches z.B. mit einer organischen Base, wie Pyridin oder 4-Methylmorpholin gebildet wird, oder ein Metallsalz, z.B. ein Alkalimetallsalz, z.B. Natriumsalz, mit einem geeigneten Derivat einer anderen Säure, beispielsweise einem Säurehalogenid, einer unsubstituierten oder durch Halogen oder Cyan substituierten Niederalkancarbonsäure, z.B. Cyanacetylchlorid, dem Halbester eines Kohlensäuremonohalogenids, z.B. Chlorameisensäureäthylester, oder -isobutylester, oder mit dem Halogenid einer Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung bei der Acylierungsreaktion verwenden.

b) Cyclisierung der Verbindung der Formel V

Die Gruppe $X^{\oplus}$ im Ausgangsmaterial der Formel V ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

In Phosphonio-Verbindungen der Formel V wird die negative Ladung durch die positiv geladene Phosphoniogruppe neutralisiert. In Phosphono-Verbindungen der Formel V wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, Dioxan oder Tetrahydrofuran, einem Carbonsäureamid, z.B. Dimethylformamid, einem Diniederalkylsulfoxid, z.B. Dimethylsulfoxid, oder einem Niederalkanol, z.B. Aethanol, oder in einem Gemisch davon, und, falls notwendig, in einer Inertgas-, z.B. Stickstoffatmosphäre, durchgeführt.

<u>c) Cyclisierung der Verbindung der Formel VI</u>

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten

Kohlenwasserstoff, z.B. Methylenchlorid oder Chloroform, bei einer Temperatur von etwa 20° bis 80°C, bevorzugt bei etwa 40° bis etwa 60°C, durchgeführt, wobei man 1 Moläquivalent einer Verbindung der Formel VI mit 2 Moläquivalenten der Phosphorverbindung umsetzt. Vorzugsweise legt man die Verbindung der Formel VI in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel VI wie weiter unten angegeben her und setzt es ohne Isolierung aus dem Reaktionsgemisch mit der organischen Verbindung des dreiwertigen Phosphors um, wobei die Endprodukte der Formel I entstehen.

d) Eliminierung des Restes X aus Verbindungen der Formel VII

Bei den Verbindungen der Formel VII handelt es sich um entsprechend substituierte 2-Thia-3-cephem-4-carbonsäuren oder insbesondere um 2-Thia-3-cephem-4-carbonsäure-1,1-dioxide.

Die Eliminierung von Schwefel aus einer 2-Thia-3-cephem-4-carbonsäure der Formel VII (X=S) erfolgt beispielsweise mit einer organischen Verbindung des dreiwertigen Phosphors, wie z.B. einer der unter Verfahren c) genannten Verbindungen. Geeignete Verbindungen des dreiwertigen Phosphors sind z.B. Triniederalkylphosphite, z.B. Triäthylphosphit, und insbesondere Triphenylphosphin. Die Eliminierung wird bei Raumtemperatur oder leicht erhöhter Temperatur, z.B. bei etwa 20° bis etwa 60°C, in einem inerten Lösungsmittel oder Lösungsmittelgemisch, z.B. in einem Kohlenwasserstoff, z.B. Benzol, einem chlorierten Kohlenwasserstoff, z.B. Chloroform, Acetonitril oder einem Niederalkanon, z.B. Aceton, wenn nötig in einer Inertgas-, wie Stickstoffatmosphäre, durchgeführt.

Die Eliminierung von Schwefeldioxid aus einer Verbindung der Formel VII, worin X für $SO_2$ steht, erfolgt beispielsweise in einer diese Verbindung enthaltenden Lösung durch mildes bis mässiges

Erwärmen, z.B. auf etwa 30° bis etwa 80°C, wobei als Lösungsmittel insbesondere inerte Lösungsmittel, wie z.B. gegebenenfalls chlorierte Kohlenwasserstoffe, z.B. Chloroform, aliphatische Aether oder aromatische Kohlenwasserstoffe, z.B. Benzol, in Frage kommen.

Bevorzugt werden solche Ausgangsmaterialien der Formeln II - VIII verwendet, die zu den eingangs als besonders bevorzugt genannten Verbindungen der Formel I führen.

In einer erhältlichen Verbindung der Formel I, worin eine oder mehrere funktionelle Gruppen geschützt sind, können diese, z.B. geschützte Carboxyl-, Hydroxy-, Amino-, und/oder Sulfogruppe, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ und/oder $R_4$ eine geschützte Carboxylgruppe bedeutet und/oder worin der Rest $R_4$ und/oder der Rest $R_6$ geschütztes Carboxyl als Substituenten enthält, kann die geschützte Carboxylgruppe in an sich bekannter Weise freigesetzt werden. So kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz,

z.B. Chrom-II-chlorid, üblicherweise in Gegenwart eines Wasserstoffabgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, in Gegenwart von Triphenylphosphin und unter Zusatz einer Carbonsäure, z.B. 2-Aethylhexansäure, oder eines Salzes davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, während Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

Andererseits können auch Verbindungen der Formel I, worin $R_2$ Carboxyl, bedeutet, in Verbindungen der Formel I überführt werden, worin $R_2$ eine geschützte Carboxylgruppe, insbesondere eine veresterte Carboxylgruppe, oder eine unter physiologischen Bedingungen spaltbare veresterte Carboxylgruppe darstellt. So kann man die freie Carboxylgruppe z.B. durch Behandeln mit einer geeigneten Diazoverbindung, wie einem Diazoniederalkan, z.B. Diazomethan, oder einem Phenyldiazoniederalkan, z.B. Diphenyldiazomethan, wenn notwendig, in Gegenwart einer Lewis-Säure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylester, wie Chlorameisensäureäthyl- oder Chlorameisensäureisobutylester, oder mit Halogenessigsäurehalogeniden, wie Trichloressigsäurechlorid) durch Umsetzen mit geeigneten Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe kann diese in eine andere veresterte Carboxylgruppe übergeführt werden, z.B. 2-Chloräthoxycarbonyl oder 2-Bromäthoxycarbonyl durch Behandeln mit einem Jodsalz, z.B. Natriumjodid, in 2-Jodäthoxycarbonyl. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähige Ester

eines entsprechenden Alkohols in eine Verbindung der Formel I überführen, worin $R_2$ eine unter physiologischen Bedingungen spaltbare veresterte Carboxylgruppe darstellt.

In verfahrensgemäss erhältlichen Verbindungen der Formel I, worin der Rest $R_1$ und/oder gegebenenfalls der Rest $R_4$ und/oder $R_6$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silyl- oder Stannylgruppe geschützte Hydroxygruppe wie eine entsprechend geschützte Aminogruppe freigesetzt (s.u.); eine Triniederalkylsilylgruppe z.B. auch mit Tetrabutylammoniumfluorid und Essigsäure (unter diesen Bedingungen werden durch trisubstituiertes Silyläthoxy geschützte Carboxygruppen nicht gespalten). Eine 2-Halogenniederalkylgruppe und eine gegebenenfalls substituierte Benzylgruppe werden reduktiv abgespalten.

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer

Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder eines Salzes davon gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel, oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

Eine geschützte, insbesondere veresterte, Sulfogruppe wird analog einer geschützten Carboxylgruppe freigesetzt.

In Verbindungen der Formel I kann man weiterhin einen Rest $R_3$ und/oder $R_4$ und/oder $R_5$ und/oder $R_6$ in einen anderen Rest $R_3$ und/oder $R_4$ und/oder $R_5$ und/oder $R_6$ überführen.

So kann beispielsweise in Verbindungen der Formel I, worin der Rest $R_4$ und/oder $R_6$ durch eine Carboxylgruppe substituiert ist, diese Carboxylgruppe nach an sich bekannten Verfahren in eine funktionell abgewandelte Carboxylgruppe, wie z.B. in eine veresterte Carboxylgruppe oder in gegebenenfalls substituiertes Carbamoyl, überführt werden. Beispielsweise erhält man durch Umsetzen einer Verbindung der Formel I, worin $R_4$ ein durch Carboxyl substituierter organischer Rest ist, mit einem Alkohol, insbesondere einem Niederalkanol, eine Verbindung der Formel I, worin $R_4$ ein durch verestertes Carboxyl, insbesondere Niederalkoxycarbonyl, substituierter organischer Rest ist, wobei man vorzugsweise in Gegenwart eines geeigneten Kondensationsmittels, z.B. eines Carbodiimids, arbeitet oder das entstehende Wasser durch azeotrope Destillation entfernt. Andererseits kann man Carboxylgruppen an Resten $R_4$ und/oder $R_6$ auch in reaktionsfähige funktionelle Derivate, wie gemischte Anhydride, z.B. Säurehalogenide, oder aktivierte Ester, überführen und diese durch Umsetzen mit einem Alkohol, z.B. Niederalkanol, Ammoniak oder einem primären oder sekundären Amin, z.B. einem Niederalkyl- oder Diniederalkylamin, in entsprechend veresterte oder amidierte Carboxylgruppen umwandeln, wobei man bei Verwendung von gemischten Anhydriden vorzugsweise in Gegenwart eines säurebindenden Mittels, wie eines aromatischen oder tertiären Amins oder eines Alkalimetall- oder Erdalkalimetallcarbonats, arbeitet.

Weist eine (Hetero)aryl-Gruppe im Rest $R_4$ oder $R_6$ eine Hydroxygruppe auf, so lässt sich diese auf üblichem Wege veräthern. Die Umsetzung zu den entsprechenden Niederalkyl-(hetero)aryl-äthern erfolgt beispielsweise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden, oder mit Diazoniederalkanen, oder, in Gegenwart eines Dehydratisierungsmittels, beispielsweise Dicyclohexylcarbodiimid, mit Hilfe von Niederalkanolen. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie einem Anhydrid davon,

z.B. dem symmetrischen Anhydrid davon oder einem gemischten Anhydrid mit einer Halogenwasserstoffsäure, erforderlichenfalls in Gegenwart eines basischen Kondensationsmittels, wie eines Alkalimetallhydroxids oder -carbonats, oder einer Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierte Hydrolyse, z.B. in Gegenwart von Natriumhydroxid.

In Verbindungen der Formel I, worin $R_4$ ein durch Amino substituierter organischer Rest bedeutet, kann die Aminogruppe in eine substituierte Aminogruppe, z.B. eine Niederalkylamino-, Diniederalkylamino-, Niederalkylenamino- oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali- oder Erdalkalimetalls oder einer heteroaromatischen Stickstoffbase, z.B. Pyridin. In analoger Weise kann Amino durch Behandeln mit einem Niederalkylendihalogenid oder -disulfonat in Niederalkylenamino und durch Behandeln mit dem reaktionsfähigen funktionellen Derivat einer Niederalkancarbonsäure, z.B. dem entsprechenden Carbonsäurehalogenid, in Niederalkanoylamino umgewandelt werden. Verbindungen der Formel I mit einem substituierbaren Ringstickstoffatom im Rest $R_4$ können auf die gleiche Weise in Verbindungen der Formel I überführt werden, welche im Rest $R_4$ ein durch einen gegebenenfalls substituierten Niederalkylrest substituiertes Ringstickstoffatom enthalten. Die mit Hilfe der genannten Umsetzungen herstellbaren neuen Verbindungen der Formel I können daher im Rest $R_4$ ein entsprechend substituiertes sekundäres, tertiäres oder auch quaternäres, d.h. positiv geladenes, Stickstoffatom enthalten.

In analoger Weise können Verbindungen der Formel I, worin $R_3$ und/oder $R_5$ und/oder $R_6$ Wasserstoff bedeutet, in Verbindungen der Formel I überführt werden, worin $R_3$ und/oder $R_5$ und/oder $R_6$ Nieder-

alkyl darstellt, oder $R_4$ zusammen mit $R_5$ für Niederalkylen steht, wobei man im Falle von $R_3$ auch eine stärkere Base als bei den Umwandlungen von $R_4$ angegeben, z.B. Natriumhydrid oder Natriumamid, anwenden kann.

Verbindungen der Formel I, worin $R_4$ und $R_5$ Wasserstoff ist, können durch Umsetzen mit einem ω-Halogenniederalkanoylhalogenid auch in Verbindungen der Formel I umgewandelt werden, worin $R_4$ und $R_5$ zusammen für durch Oxo substituiertes Niederalkylen stehen. Weiterhin können Verbindungen der Formel I, worin $R_6$ Wasserstoff ist, durch Umsetzen mit einer den Acylrest einer Carbonsäure, eines Halbesters der Kohlensäure, einer gegebenenfalls substituierten Carbaminsäure oder Thiocarbaminsäure, Sulfonsäure oder Amidosulfonsäure einführenden Verbindung, z.B. einem Säurehalogenid einer solchen Säure, z.B. dem Chlorid oder Bromid davon, vorzugsweise in Gegenwart eines säurebindenden Mittels, wie eines der oben genannten, in Verbindungen der Formel I überführt werden, worin $R_6$ Acyl ist.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der α-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physikalisch-chemischen Methoden (z.B. fraktioniertes Kristallisieren, Adsorptionschromatographie) trennen und die einzelnen diastereomeren Verbindungen dann in die optisch aktiven Verbindungen spalten. Zur Trennung in Antipoden besonders geeignete Racemate sind solche, die eine saure Gruppe besitzen, wie z.B. Racemate von Verbindungen der Formel I, worin $R_2$ Carboxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, (+)- oder (-()-2-Octanol verestert werden, worauf nach erfolgter Isolierung des gewünschten Diastereomeren die Carboxylgruppe freigesetzt wird.

Zur Racemattrennung kann auch eine vorhandene Hydroxygruppe mit optisch aktiven Säuren oder deren reaktionsfähigen, funktionellen Derivaten verestert werden, wobei sich diastereomere Ester bilden. Solche Säuren sind beispielsweise (-)-Abietinsäure, D(+)- und L(-)-Aepfelsäure, N-acylierte optisch aktive Aminosäuren, (+)- und (-)-Camphansäure, (+)- und (-)-Ketopinsäure, L(+)-Ascorbinsäure, (+)-Camphersäure, (+)-Campher-10-sulfonsäure(ß), (+)- oder

(-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-Benzoyl- und Di-O-p-Tolylderivate.

Durch Umsetzung mit optisch aktiven Isocyanaten, wie mit (+)- oder (-)-1-Phenyläthylisocyanat, können Verbindungen der Formel (I), worin $R_2$ geschütztes Carboxy und $R_1$ durch Hydroxy substituiertes Niederalkyl bedeutet, in ein Gemisch diastereomerer Urethane umgewandelt werden.

Basische Racemate, z.B. Verbindungen der Formel I, worin der Rest $R_4$ oder $R_6$ durch Amino substituiert ist oder worin wenigstens einer der Reste $R_5$ und $R_6$ Wasserstoff bedeuten, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Adsorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material, wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produktes aus.

Die Auftrennung der Racemate in die optischen Antipoden kann auf einer beliebigen Verfahrensstufe, d.h. z.B. auch auf der Stufe der Ausgangsverbindungen der Formeln II, V, VI oder VII oder auf einer beliebigen Stufe der nachstehend beschriebenen Verfahren zur Herstellung der Ausgangsverbindungen durchgeführt werden.

Bei allen nachträglichen Umwandlungen erhaltener Verbindungen der Formel I werden solche Reaktionen bevorzugt, die unter schwach alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formel II können beispielsweise hergestellt werden, indem man ein Phosphoran der Formel

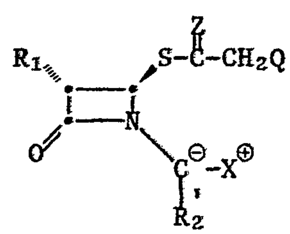

(IXa)

oder

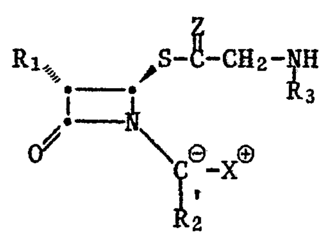

(IXb)

in analoger Weise, wie unter Verfahren b) beschrieben, ringschliesst.

Die Ausgangsverbindungen der Formel IV, IXa und IXb sind bekannt oder können nach bekannten Methoden hergestellt werden.

Die Ausgangsverbindungen der Formeln V und VI können, wie im folgenden Reaktionsschema I dargestellt, hergestellt werden:

Reaktionsschema I

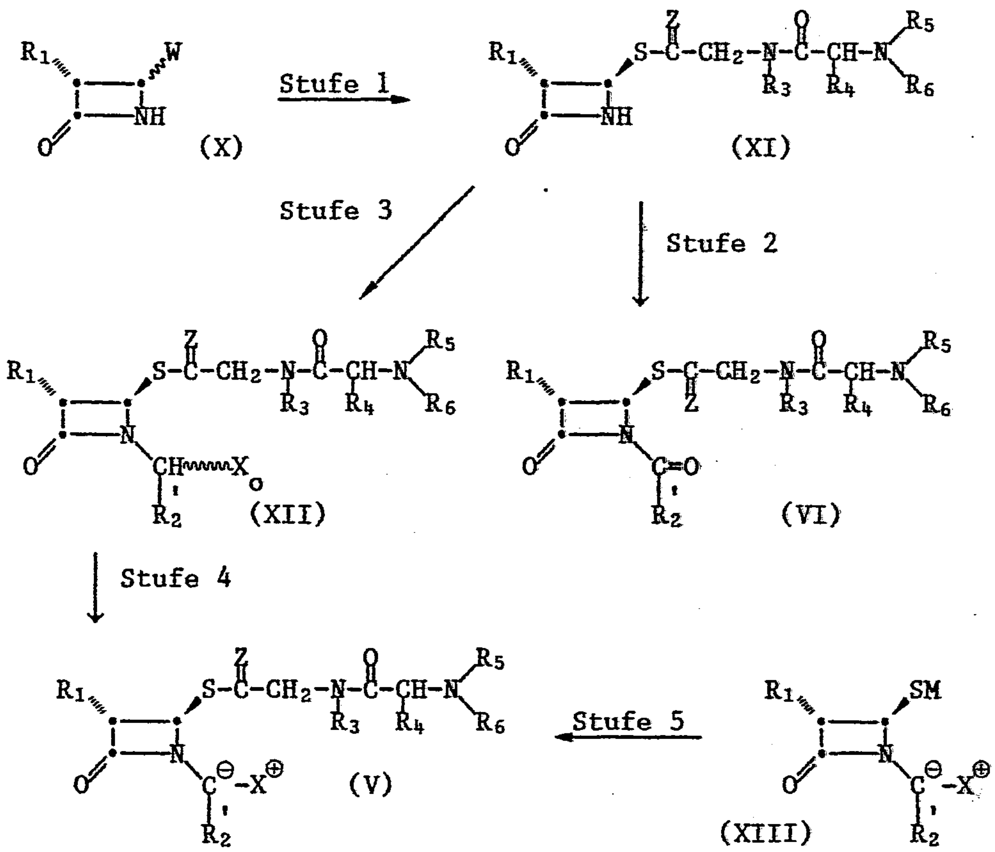


(X) (XI) (XII) (VI) (V) (XIII)

Stufe 1

Ein Thioazetidinon der Formel XI wird erhalten, indem man eine Verbindung der Formel X mit einer den Rest

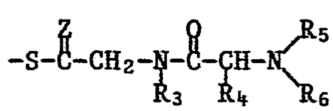

einführenden Verbindung umsetzt.

In einem Ausgangsmaterial der Formel X ist W ein nucleofuger, durch die Gruppe

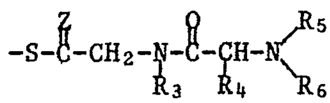

ersetzbarer Rest. Solche Reste W sind beispielsweise Acyloxyreste, Sulfonylreste $R_o-SO_2-$, worin $R_o$ ein organischer Rest ist, Azido oder Halogen. In einem Acyloxyrest W ist Acyl z.B. der Rest einer organischen Carbonsäure und bedeutet beispielsweise Niederalkanoyl, z.B. Acetyl oder Propionyl, gegebenenfalls z.B. durch Nitro substituiertes Benzoyl, z.B. Benzoyl oder 2,4-Dinitrobenzoyl, oder Phenylniederalkanoyl, z.B. Phenylacetyl. In einem Sulfonylrest $R_o-SO_2-$ ist $R_o$ beispielsweise gegebenenfalls durch Hydroxy substituiertes Niederalkyl, wie Methyl, Aethyl, 2-Hydroxyäthyl, 1-Hydroxyprop-2-yl oder 1-Hydroxy-2-methyl-prop-2-yl, Benzyl oder gegebenenfalls z.B. durch Niederalkyl oder Halogen substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Eine den Rest

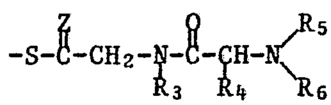

einführende Verbindung ist beispielsweise eine Säure der Formel

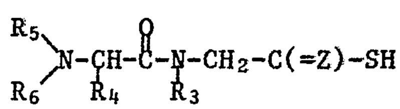

oder insbesondere ein Salz, z.B. ein Alkalimetallsalz, wie das Natrium- oder Kaliumsalz, davon. Die Substitution kann in einem organischen Lösungsmittel, wie in einem Niederalkanol, z.B. Methanol

oder Aethanol, einem Niederalkanon, z.B. Aceton, einem Niederalkancarbonsäureamid, z.B. Dimethylformamid, einem cyclischen Aether, z.B. Tetrahydrofuran oder Dioxan, oder in einem ähnlichen inerten Lösungsmittel durchgeführt werden. Die Reaktion wird üblicherweise bei Raumtemperatur durchgeführt, kann aber auch bei erhöhter oder erniedrigter Temperatur, z.B. bei etwa 0° bis etwa 40°C, durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

Die eintretende Gruppe wird von dem Rest $R_1$ bevorzugt in die trans-Stellung dirigiert. Daher können sowohl (3S,4R)- als auch (3S,4S)-konfigurierte Ausgangsverbindungen der Formel X eingesetzt werden. Obwohl die trans-Isomeren überwiegend gebildet werden, können doch gelegentlich auch geringe Mengen des cis-Isomeren entstehen. Die Abtrennung der cis-Isomeren erfolgt, wie oben beschrieben, nach konventionellen Methoden, insbesondere durch Chromatographie und/oder durch Kristallisation.

Geeignete Ausgangsverbindungen der Formel X sind beispielsweise aus der Europäischen Patentanmeldung Nr. 82113, der Deutschen Offenlegungsschrift Nr. 3 224 055 oder der Deutschen Offenlegungsschrift 3 013 997 bekannt oder können in analoger Weise hergestellt werden. Sie können auch nach den in den Beispielen beschriebenen Verfahren hergestellt worden.

Stufe 2

Eine Ausgangsverbindung der Formel (VI) wird erhalten, indem man ein Azetidinon der Formel (XI) mit einer Säure der Formel $R_2'$-COOH oder insbesondere einem reaktionsfähigen Derivat, wie einem Ester oder Säurehalogenid, z.B. dem Säurechlorid, davon bei einer Temperatur von 20° bis 80°C, bevorzugt bei 40° bis 60°C, in einem inerten Lösungsmittel, wie einem der bei der Umsetzung von Verbindungen der Formel VI zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall- oder Erdalkalimetallcarbonats oder -hydrogencarbonats, z.B. Kaliumcarbonat oder Calciumcarbonat.

Stufe 3

Verbindungen der Formel XII, worin $X_o$ für eine reaktionsfähige veresterte Hydroxygruppe, insbesondere für Halogen, z.B. Chlor oder Brom, oder organisches Sulfonyloxy, z.B. Niederalkansulfonyloxy, wie Methansulfonyloxy, oder Arensulfonyloxy, z.B. Benzol- oder 4-Methylbenzolsulfonyloxy, steht, werden hergestellt, indem man eine Verbindung der Formel XI mit einer Glyoxylsäure-Verbindung der Formel $OHC-R_2'$ oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel XII, worin $X_o$ für Hydroxy steht, die Hydroxygruppe in eine reaktionsfähige veresterte Hydroxygruppe umwandelt.

Die Verbindungen der Formel XII werden üblicherweise als Gemisch der beiden Isomeren [bezüglich der Gruppierung $-CH(R_2') \sim X^o$] erhalten. Man kann aber auch die reinen Isomeren davon isolieren, z.B. durch Chromatographie.

Die Anlagerung der Glyoxylsäureester-Verbindung an das Stickstoffatom des Lactamrings in der Verbindung der Formel XI findet bei Raumtemperatur oder, falls notwendig, unter Erwärmen, z.B. bei etwa 40° bis etwa 100°C, und zwar in Abwesenheit eines eigentlichen Kondensationsmittels statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels, wie eines Molekularsiebs, enfernt wird. Vorzugsweise arbeitet man in Gegenwart eines geeigneten Lösungsmittels, wie z.B. Dioxan, Toluol oder Dimethylformamid, oder Lösungsmittelgemisches, und, wenn erwünscht oder notwendig in der Atmosphäre eines Inertgases, wie Stickstoff.

Die Ueberführung einer Hydroxygruppe $X_o$ in eine reaktionsfähige veresterte Hydroxygruppe $X_o$ in einer Verbindung der Formel XII wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphoniumdibromid oder -dichlorid oder einem geeigneten organischen Sulfonsäurehalogenid, wie -chlorid, vorzugsweise in Gegenwart eines basischen, in erster Linie organischen basischen Mittels, wie eines aliphatischen tertiären Amins, z.B. Triäthylamin oder Diisopropyläthylamin, oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, bei Raumtemperatur oder wenn notwendig unter Kühlen, z.B. bei etwa -30° bis etwa 30°C, und gegebenenfalls in der Atmosphäre eines Inertgases, wie Stickstoff.

Stufe 4

Das Ausgangsmaterial der Formel (V) wird erhalten, indem man eine Verbindung der Formel (XII) mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt.

Die Umwandlung der Verbindung der Formel (XII) in die Verbindung der Formel (V) wird vorzugsweise in Gegenwart eines geeigneten inerten Lösungsmittels, wie eines Kohlenwasserstoffs, z.B. Cyclohexan oder Benzol, oder eines Aethers, z.B. Dioxan, oder eines Lösungsmittelgemisches vorgenommen. Je nach Reaktionsfähigkeit arbeitet man unter Kühlen oder bei erhöhter Temperatur, etwa zwischen -10° und +100°C, bevorzugt bei etwa 20° bis 80°C, und, falls erforderlich, in der

Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man üblicherweise in Gegenwart eines basischen Mittels, wie einer organischen Base, z.B. eines Amins, wie Triäthylamin, Diisopropyläthylamin, Pyridin, Lutidin, oder "Polystyrol-Hünigbase", oder einer anorganischen Base, z.B. eines Alkalimetallcarbonats, z.B. Natrium- oder Kaliumcarbonat, wobei das primär entstandene Phosphoniumsalz der Formel

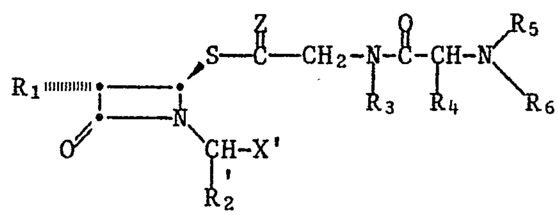

(Va) ,

worin X' eine Phosphonogruppe oder eine Phosphoniogruppe zusammen mit einem Anion, je nach Bedeutung des Restes $X_o$ z.B. das Chlorid-Anion, bedeutet, in das Ylid-Ausgangsmaterial der Formel V umgewandelt wird. Man kann aber auch in Abwesenheit einer Base arbeiten und eine Verbindung der Formel (Va), insbesondere eine entsprechende Phosphono-Verbindung, isolieren und diese bei der Herstellung der Endprodukte der Formel (I) in situ in das Ausgangsmaterial der Formel V überführen.

Stufe 5

Eine Verbindung der Formel (V) kann weiterhin erhalten werden, indem man ein Mercaptid der Formel (XIII), worin M für ein Metallkation steht, mit einem den Rest

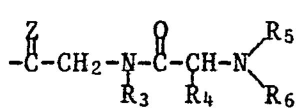

einführenden Acylierungsmittel behandelt.

In dem Ausgangsmaterial der Formel (XIII) ist das Metallkation M beispielsweise ein Kation der Formel $M^+$ oder $M^{2+}/2$, wobei $M^+$ insbesondere für ein Silberkation und $M^{2+}$ insbesondere für das zweiwertige Kation eines geeigneten Uebergangmetalls, z.B. Kupfer, Blei oder Quecksilber, steht.

Ein den Rest

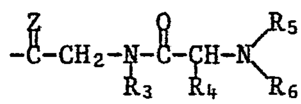

einführendes Acylierungsmittel ist z.B. die Säure

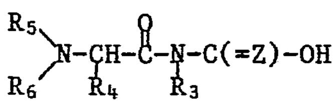

(XIV)

oder insbesondere ein reaktionsfähiges funktionelles Derivat, wie ein Säurehalogenid, z.B. Chlorid oder Bromid, Azid oder Anhydrid davon.

Die Acylierung erfolgt, wenn ein reaktionsfähiges funktionelles Derivat der Säure der Formel XIV, z.B. das Säurechlorid, eingesetzt wird, in einem inerten Lösungsmittel, wie einem chlorierten Kohlenwasserstoff, z.B. Methylenchlorid, oder einem Aether, z.B. Diäthyläther oder Dioxan, bei Raumtemperatur oder unter Erhitzen oder Kühlen, z.B. in einem Temperaturbereich von ca. -50° bis ca. +60°C, insbesondere bei ca. -30° bis ca. +20°C.

Die Ausgangsverbindungen der Formel (XIII) können beispielsweise hergestellt werden, indem man ein Azetidinon der Formel

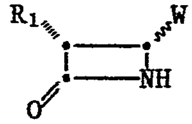

(X)

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

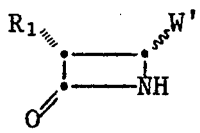

(XV)

überführt, worin W' Triphenylmethylthio oder Niederalkanoylthio, z.B. Acetylthio, bedeutet, diese analog dem in den Reaktionsstufen 3 und 4 beschriebenen Verfahren in eine Verbindung der Formel

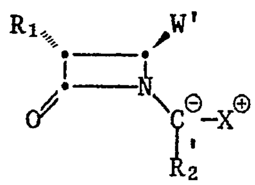

(XVI)

überführt und diese in Gegenwart einer Base, z.B. Pyridin oder Tri-n-butylamin, in einem geeigneten Lösungsmittel, z.B. Diäthyläther oder Methanol, mit einem Salz der Formel MA, worin M die obige Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt.

Die Ylide der Formel V können direkt in die Cyclisierungsreaktion zur Herstellung der Endprodukte der Formel I eingesetzt werden. Man kann aber auch in Verbindungen der Formel V, worin $R_1$ durch eine geschützte Hydroxygruppe, z.B. eine hydrolytisch leicht spaltbare geschützte Hydroxygruppe, wie trisubstituiertes Silyloxy, substituiertes Niederalkyl ist, zuerst die Hydroxyschutzgruppe abspalten und dann die erhaltene Verbindung der Formel V, worin $R_1$ durch Hydroxy substituiertes Niederalkyl ist, in die Cyclisierungsreaktion einsetzen.

Die Ausgangsverbindungen der Formel VII, worin X für die Gruppe -S-steht, sind bekannt (beispielsweise aus dem belgischen Patent-898382 und aus Tetrahedron Letters <u>1983</u>, 1631 - 1634) oder können in analoger Weise hergestellt werden. Verbindungen der Formel VII, worin X für eine Sulfonylgruppe $-SO_2-$ steht, werden hergestellt, indem man eine Verbindung der Formel

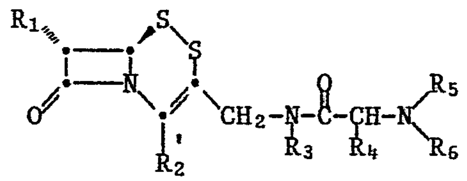

(VIIa)

worin $R_1$, $R_3$, $R_4$, $R_5$ und $R_6$ die unter Formel I angegebenen Bedeutungen haben und $R_2'$ geschütztes Carboxyl ist, mit einer aliphatischen oder aromatischen Percarbonsäure, z.B. Peressigsäure, m-Chlorperbenzoesäure oder Monoperphthalsäure, in einem inerten Lösungsmittel oder Lösungsmittelgemisch, z.B. in einem Halogenkohlenwasserstoff, z.B. Chloroform, einem Aether oder einem aromatischen Kohlenwasserstoff, z.B. Benzol, bei einer Temperatur von etwa 0° bis etwa 60°C umsetzt.

Die Startverbindungen der Formel VIII sind bekannt oder können nach bekannten Verfahren hergestellt werden.

Die Startverbindungen der Formeln IXa und IXb können hergestellt werden, indem man eine Verbindung der Formel X mit einer Thiosäure der Formel $Q-CH_2-C(=Z)-SH$ oder $R_3-NH-CH_2-C(=Z)-SH$ umsetzt und die entstandenen Azetidinone der Formeln

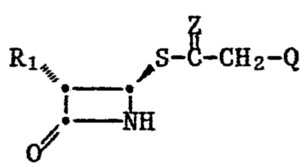

(IXc)

oder

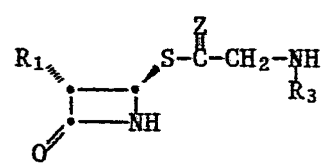

(IXd)

gemäss den im Reaktionsschema I unter den Stufen 3 und 4 beschriebenen Verfahren weiter umsetzt.

In den Verbindungen der Formeln II, V - XVI können vorhandene funktionelle Gruppen in geschützte funktionelle Gruppen oder vorhandene geschützte funktionelle Gruppen in die freien oder in anders geschützte Gruppen überführt werden. Weiterhin kann man in Verbindungen der Formeln V, VI, VII, XI und XII einen Rest $R_4$ in einen anderen Rest $R_4$ umwandeln. Bei diesen Umwandlungen können unter Berücksichtigung der weiteren in den Molekülen enthaltenen Substituenten die gleichen Methoden zur Anwendung gelangen, wie bei den entsprechenden Umwandlungen in die Verbindungen der Formel (I) angegeben ist.

Man kann die beschriebenen Verfahren zur Herstellung der Verbindungen der Formeln II, V - XVI sowie die angegebenen Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat, wie oben beschrieben, die optisch aktiven Verbindungen gemäss der vorliegenden Erfindung isolieren.

Die Erfindung betrifft ebenfalls die neuen Ausgangsverbindungen sowie verfahrensgemäss erhältliche neue Zwischenprodukte, wie diejenigen der Formeln V - VII, XI und XII, sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren

Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 1 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm
IR: Infrarotspektrum
UV: Ultraviolettspektrum
THF: Tetrahydrofuran

## Experimenteller Teil

Beispiel 1: (5R,6S)-2-[N-(4-Nitrobenzyloxylcarbonyl)-glycylaminomethyl]-6-tert.butyldimethylsilyloxmethyl-2-penem-3-carbonsäure-4-nitrobenzylester

1,52 g 2-[(3S,4R)-3-tert.Butyldimethylsilyloxymethyl-4-[N-(4-nitrobenzyloxycarbonyl)-glycyl]-aminoacetylthio]-2-oxo-azetidin-1-yl]-2-triphenylphosphoranylidenessigsäure-4-nitrobenzylester werden in 300 ml absolutem Toluol während 2,5 Stunden bei Rückflusstemperatur und unter Argonatmosphäre gerührt. Nach Abkühlen wird das Lösungsmittel abdestilliert und der Rückstand wird an Silicagel chromatographiert. (Laufmittel Toluol/Aethylacetat 4:1).
DC (Silicagel/Aethylacetat) $R_f$ = 0,5
IR (Methylenchlorid) 3430; 1785; 1715; 1695 $cm^{-1}$.

Das Ausgangsmittel wird wie folgt hergestellt:

a.) N-(4-Nitrobenzyloxycarbonyl)-glycyl-glycin

13,2 g Glycyl-glycin werden in 22 ml Wasser und 45 ml 5N NaOH gelöst, auf 0° gekühlt und mit 23,7 g Chlorameisensäure-p-nitrobenzylester versetzt. Das Reaktionsgemisch wird dann weitere 30 Minuten bei 0° und anschliessend 19 Stunden bei Raumtemperatur

gerührt. Dann wird filtriert, das Filtrat mit 200 ml Wasser verdünnt und zweimal mit Methylenchlorid gewaschen. Das Reaktionsgemisch wird mit 4N Salzsäure sauer gestellt und dreimal mit Aethylacetat extrahiert. Die vereinigten organischen Phasen werden mit konzentrierter NaCl-Lösung (Sole) gewaschen, über Magnesiumsulfat getrocknet und eingeengt. Das Rohprodukt wird aus Wasser umkristallisiert. Smp.: 171°.
IR (Tetrahydrofuran): 1730; 1685 $cm^{-1}$.

b.) <u>N-(4-Nitrobenzyloxycarbonyl)-glycyl-thioglycin</u>
22,35 g N-(4-Nitrobenzyloxycarbonyl)-glycyl-glycin werden in 240 ml absolutem Tetrahydrofuran gelöst, mit 11,5 ml Triäthylamin versetzt und auf -15° abgekühlt. Nach Zugabe von 7,53 ml Chlorameisensäureäthylester wird die weisse Suspension 20 Minuten nachgerührt. Nach Zugabe von weiteren 11,5 ml Triäthylamin wird während 3 Stunden Schwefelwasserstoff eingeleitet, wobei man die Temperatur langsam auf Raumtemperatur steigen lässt. Das überschüssige $H_2S$ wird durch Einblasen von Stickstoff entfernt. Das Ungelöste wird abfiltriert und das Filtrat eingeengt. Nach Aufnahme des Rückstandes in Wasser wird mit 2N Salzsäure angesäuert und mehrmals mit Aethylacetat extrahiert. Die vereinten organischen Extrakte werden mit Sole gewaschen, über Magnesiumsulfat getrocknet und eingeengt.
IR (Tetrahydrofuran): 2460; 1725; 1690 $cm^{-1}$.

c.) <u>(3S,4R)-3-(tert.Butyl-dimethylsilyloxymethyl)-4-[N-(4-nitrobenzyloxycarbonyl)-glycyl-aminoacetylthio]-azetidin-2-on</u>
3,4 g (3S,4R)-3-tert.Butyl-dimethylsilyloxymethyl-4-methylsulfonylazetidin-2-on (Europäische Patentanmeldung Nr. 82113) und 5,3 g N-(4-Nitrobenzyloxycarbonyl)-glycyl-thioglycin werden in 85 ml Methylenchlorid gelöst und mit 85 ml Wasser und 17,4 ml 1N NaOH sehr kräftig gerührt. Nach 2 Stunden wird die organische Phase abgetrennt und die wässrige noch zweimal mit Methylenchlorid nachextrahiert. Die vereinten Extrakte werden mit wässriger $NaHCO_3$ Lösung und Sole gewaschen, über Magnesiumsulfat getrocknet und eingedampft. Das Rohprodukt wird durch Chromatographie an Silicagel (Laufmittel Aethylacetat) gereinigt.

DC (Silicagel; Aethylacetat) $R_f$ = 0,36.
IR (Methylenchlorid) 3420; 1775; 1725; 1685 $cm^{-1}$.

d.) 2-[(3S,4R)-3-tert.Butyldimethylsilyloxymethyl-4-[N-(4-nitrobenzyloxycarbonyl)-glycyl-aminoacetylthio]-2-oxo-azetidin-1-yl]-2-hydroxyessigsäure-4-nitrobenzylester

7,6 g (3S,4R)-3-tert.Butyl-dimethylsilyloxymethyl-4-[N-(4-nitrobenzyloxycarbonyl)-glycyl-aminoacetylthio]-azetidin-2-on und 7,17 g Glyoxylsäure-4-nitrobenzylester-äthylhemiacetal in 140 ml absolutem Benzol werden mit 27 g Molekularsieb (4 Å) versetzt und während 8 Stunden unter Rückfluss gerührt. Nach Abfiltrieren und Einengen am Rotationsverdampfer unter vermindertem Druck wird das Rohprodukt durch Chromatographie an Silicagel gereinigt (Laufmittel Toluol-Aethylacetat 1:1 bis Aethylacetat).
DC (Silicagel/Aethylacetat) $R_f$ = 0,33 und 0,42
IR (Methylenchlorid): 3430; 1770; 1745; 1725; 1685 $cm^{-1}$.

e.) 2-[(3S,4R)-3-tert.Butyldimethylsilyloxymethyl-4-[N-(4-nitrobenzyloxycarbonyl)-glycyl-aminoacetylthio]-2-oxo-azetidin-1-yl]-2-triphenylphosphoranylidenessigsäure-4-nitrobenzylester

Zu einer Lösung von 0,31 g 2-[(3S,4R)-3-tert.Butyl-dimethylsilyloxymethyl-4-[N-(4-nitrobenzyloxycarbonyl)-glycyl-aminoacetylthio]-2-oxo-azetidin-1-yl]-2-hydroxyessigsäure-4-nitrobenzylester in 5 ml Tetrahydrofuran werden unter Rühren bei 0° nacheinander 44 µl Thionylchlorid und 50 µl Pyridin innert 5 Minuten zugegeben. Die weisse Suspension wird 30 Minuten bei 0° nachgerührt und über Hyflo filtriert. Nach dem Waschen des Rückstands mit Toluol wird am Rotationsverdampfer eingeengt. Der Rückstand wird in 3 ml Dioxan gelöst, mit 151 mg Triphenylphosphin und 67 µl 2,6-Dimethylpyridin versetzt und während 69 Stunden bei 40° Badtemperatur gerührt. Das Gemisch wird über Hyflo filtriert und der Rückstand mit Toluol nachgewaschen. Die vereinigten Filtrate werden eingedampft. Die Chromatographie des Rückstandes an Silicagel (Laufmittel Toluol-Aethylacetat 1:1 bis Aethylacetat) ergibt das reine Produkt.
DC (Silicagel/Aethylacetat) $R_f$ = 0,44
IR (Methylenchlorid) 3430; 1750; 1730; 1690; 1620 $cm^{-1}$.

Beispiel 2: (5R,6S) 2-[N-(4-Nitrobenzyloxycarbonyl)-(2S)-alanyl-aminomethyl]-6-tert.butyldimethylsilyloxymethyl-2-penem-3-carbonsäure-4-nitrobenzylester

Analog Beispiel 1 werden 1,55 g 2-[(3S,4R)-3-tert.Butyldimethylsilyloxymethyl-4-[N-(4-nitrobenzyloxycarbonyl)-2-(2S)-alanyl-aminoacetylthio]-2-oxo-azetidin-1-yl]-2-triphenylphosphoranylidenessigsäure-4-nitrobenzylester in die Titelverbindung überführt.

DC (Silicagel/Aethylacetat) $R_f$ = 0,56

IR (Methylenchlorid) 3420; 1782; 1715; 1695 $cm^{-1}$.

Das Ausgangsmaterial kann wie folgt hergestellt werden:

a.) N-(4-Nitrobenzyloxycarbonyl)-(2S)-alanyl-glycin

Analog Beispiel 1a) werden 10,1 g (2S)-Alanyl-glycin in die Titelverbindung überführt.

IR (Tetrahydrofuran). 1730; 1687 $cm^{-1}$.

b.) N-(4-Nitrobenzyloxycarbonyl)-(2S)-alanyl-thioglycin

Analog Beispiel 1b) werden 7,3 g N-(4-Nitrobenzyloxycarbonyl)-(2S)-alanyl-glycin in die Titelverbindung überführt.

IR (Methylenchlorid): 2570; 1715; 1685 $cm^{-1}$.

c.) (3S,4R)-3-(tert.Butyl-dimethylsilyloxymethyl)-4-[N-(4-nitrobenzyloxycarbonyl)-(2S)-alanyl-aminoacetylthio]-azetidin-2-on

Analog Beispiel 1c) werden 6,5 g N-(4-Nitrobenzyloxycarbonyl)-(2S)-alanyl-thioglycin in die Titelverbindung überführt.

DC (Silicagel; Aethylacetat) : $R_f$ = 0.43.

IR (Methylenchlorid) 3420; 1772; 1720; 1687 $cm^{-1}$.

d.) 2-[(3S,4R)-3-(tert.Butyl-dimethylsilyloxymethyl)-4-[N-(4-nitrobenzyloxycarbonyl)-(2S)-alanyl-aminoacetylthio]-2-oxo-azetidin-1-yl]-3-hydroxyessigsäure-4-nitrobenzylester

Analog Beispiel 1d) werden 5,6 g (3S,4R)-3-tert.Butyl-dimethylsilyloxymethyl-4-[N-(4-nitrobenzyloxycarbonyl)-(2S)-alanyl-aminoacetylthio]-azetidin-2-on in die Titelverbindung überführt.

DC (Silicagel/Toluol-Aethylacetat 2:3): $R_f$ = 0,16 und 0,22;
IR (Methylenchlorid) 3430; 1775; 1750; 1730; 1690 $cm^{-1}$.

e.) 2-[(3S,4R)-3-tert.Butyldimethylsilyloxymethyl-4-[N-(4-nitrobenzyloxycarbonyl)-(2S)-alanyl-aminoacetylthio]-2-oxo-azetidin-1-yl]-2-triphenylphosphoranylidenessigsäure-4-nitrobenzylester
Analog Beispiel 1e) werden 4,25 g 2-[(3S,4R)-3-tert.Butyl-dimethylsilyloxymethyl-4-[N-(4-nitrobenzyloxycarbonyl)-(2S)-alanyl-aminoacetylthio]-2-oxoazetidin-1-yl]-2-hydroxyessigsäure-4-nitrobenzylester in die Titelverbindung überführt.
DC (Silicagel/Toluol-Aethylacetat 2:3): $R_f$ = 0,27.
IR (Methylenchlorid) 3430; 1750; 1730; 1692; 1625 $cm^{-1}$.

Beispiel 3: (5R,6S)-2-[N-(4-Nitrobenzyloxycarbonyl)-glycylaminomethyl]-6-hydroxymethyl-2-penem-3-carbonsäure-4-nitrobenzylester
Eine Lösung von 1,04 g (5R,6S)-2-[N-(4-Nitrobenzyloxycarbonyl)-glycylaminomethyl]-6-tert.butyldimethylsilyloxymethyl-2-penem-3-carbonsäure-4-nitrobenzylester in 16 ml absolutem THF wird nacheinander mit 0,58 ml Essigsäure und tropfenweise mit 25,4 ml einer 0,1 M Tetrabutylammonium-fluorid-Lösung in THF versetzt. Nach 2 Stunden Rühren bei Raumtemperatur wird mit 300 ml Methylenchlorid verdünnt und nacheinander mit 50 ml gesättigter wässriger $NaHCO_3$-Lösung und Sole gewaschen. Die organischen Extrakte werden über Magnesiumsulfat getrocknet und dann eingedampft. Das Rohprodukt wird durch Chromatographie an Silicagel gereinigt (Laufmittel: Aethylacetat).
DC (Silicagel/Aethylacetat) $R_f$ = 0,2.
IR (KBr) 3460; 3270; 1780; 1730; 1695; 1670 $cm^{-1}$.

Beispiel 4: (5R,6S)-2-[N-(4-Nitrobenzyloxycarbonyl)-(2S)-alanylaminomethyl]-6-hydroxymethyl-2-penem-3-carbonsäure-4-nitrobenzylester.
Analog Beispiel 3 werden 0,95 g (5R,6S)-2-[N-(4-Nitrobenzyloxycarbonyl)-(2S)-alanylaminomethyl]-6-tert.butyldimethylsilyloxymethyl-2-penem-3-carbonsäure-4-nitrobenzylester in die Titelverbindung überführt.

DC (Silicagel/Aethylacetat) $R_f$ = 0,3.
IR (KBr) 3430; 3270; 1780; 1730; 1700; 1660 $cm^{-1}$.

Beispiel 5: (5R,6S)-2-Glycylaminomethyl-6-hydroxymethyl-2-penem-3-carbonsäure

Eine Lösung von 0,13 g (5R,6S)-2-[N-(4-Nitrobenzyloxycarbonyl)-glycylaminomethyl]-6-hydroxymethyl-2-penem-3-carbonsäure-4-nitrobenzylester in 16 ml Aethylacetat und 7 ml Tetrahydrofuran werden mit 14 ml Wasser und 0,1 g 10 % Palladium/Kohle-Katalysator versetzt. Nach einer Stunde Hydrierung bei Normaldruck und Raumtemperatur gibt man zum Gemisch 2,16 ml 0,1 N HCl und weitere 30 mg 10 % Palladium/Kohle dazu. Dann wird noch eine Stunde unter gleichen Bedingungen hydriert. Der Katalysator wird abfiltriert und mit Wasser/Aethylacetat gewaschen. Die organische Phase wird noch einmal mit Wasser extrahiert. Die vereinten wässrigen Extrakte werden neutral gestellt, mit Aethylacetat und dann Methylenchlorid gewaschen und nach Konzentrieren am Rotationsverdampfer wird das Rohprodukt auf einer XAD-2 Säule gereinigt.
DC (Reverse Phase Opti $UPC_{12}$, Wasser): $R_f$ = 0,67
UV (Wasser): $\lambda_{max}$ = 302 nm.

Beispiel 6: (5R,6S)-2-[(2S)-Alanylaminomethyl]-6-hydroxymethyl-2-penem-3-carbonsäure

Analog Beispiel 5 werden 0,44 g (5R,6S)-2-[N-(4-Nitrobenzyloxycarbonyl)-(2S)-alanylaminomethyl]-6-hydroxymethyl-2-penem-3-carbonsäure-4-nitrobenzylester in die Titelverbindung überführt.
DC (Reverse Phase Opti $UPC_{12}$, Wasser): $R_f$ = 0,61.
UV (Phosphatpuffer pH 7,4) $\lambda_{max}$ = 304 nm.

Beispiel 7: In analoger Weise, wie in den vorangehenden Beispielen 1 - 6 beschrieben, können die folgenden Verbindungen hergestellt werden:
(5R,6S)-2-[(2R)-Alanylaminomethyl]-6-hydroxymethyl-2-penem-3-carbonsäure, UV (Phosphatpuffer pH 7,4): $\lambda_{max}$ = 303 nm.
(5R,6S)-2-Glycylaminomethyl-6-[(1R)-1-hydroxyäthyl]-2-penem-3-carbonsäure, DC (Reversed phase OPTI $UPC_{12}$, Wasser): $R_f$ = 0,62;

UV (Wasser): $\lambda_{max}$ = 304 nm.
(5R,6S)-2-[(2S)-Alanylaminomethyl]-6-[(1R)-1-hydroxyäthyl]-2-penem-3-carbonsäure), DC (Reversed phase Opti $UPC_{12}$, Wasser): $R_f$ = 0,56; UV (Wasser): $\lambda_{max}$ = 305 nm.
(5R,6S)-2-[(2S)-Tyrosylaminomethyl]-6-[(1R)-1-hydroxyäthyl]-2-penem-3-carbonsäure), DC (Reversed phase, OPTI $UPC_{12}$, Wasser): $R_f$ = 0,27; UV (Wasser): $\lambda_{max}$ = 307 nm.
(5R,6S)-2-[(2R)-Tyrosylaminomethyl]-6-[(1R)-1-hydroxyäthyl]-2-penem-3-carbonsäure), UV (Wasser): $\lambda_{max}$ = 306 nm.
(5R,6S)-2-[(2S)-Serylaminomethyl]-6-hydroxymethyl-2-penem-3-carbonsäure, UV (Wasser): $\lambda_{max}$ = 303 nm.
(5R,6S)-2-[(2R)-Serylaminomethyl]-6-hydroxymethyl-2-penem-3-carbonsäure, UV (Wasser): $\lambda_{max}$ = 302 nm.
(5R,6S)-2-[(2S)-Serylaminomethyl]-6-[(1R)-1-hydroxyäthyl]-2-penem-3-carbonsäure, DC (Reversed phase, OPTI $UPC_{12}$, Wasser) $R_f$ = 0,64; UV (Wasser): $\lambda_{max}$ = 304 nm.
(5R,6S)-2-[(2R)-Serylaminomethyl]-6-[(1R)-1-hydroxyäthyl]-2-penem-3-carbonsäure, DC (Reversed phase, OPTI $UPC_{12}$, Wasser) $R_f$ = 0,60; UV (Wasser) $\lambda_{max}$ = 303 nm.
(5R,6S)-2-[(2S)-Histidylaminomethyl]-6-hydroxymethyl-2-penem-3-carbonsäure, UV (Wasser): $\lambda_{max}$ = 304 nm.
(5R,6S)-2-[(2R)-Histidylaminomethyl]-6-hydroxymethyl-2-penem-3-carbonsäure, UV (Wasser): $\lambda_{max}$ = 303 nm.
(5R,6S)-2-[(2S)-Histidylaminomethyl]-6-[(1R)-1-hydroxyäthyl]-2-penem-3-carbonsäure, UV (Wasser): $\lambda_{max}$ = 303,5 nm.
(5R,6S)-2-[(2R)-Histidylaminomethyl]-6-[(1R)-1-hydroxyäthyl]-2-penem-3-carbonsäure, UV (Wasser): $\lambda_{max}$ = 304 nm.
(5R,6S)-2-[(2S)-Threonylaminomethyl]-6-[(1R)-1-hydroxyäthyl]-2-penem-3-carbonsäure, DC (Reversed phase, OPTI $UPC_{12}$, Wasser): $R_f$ = 0,29; UV (Wasser): $\lambda_{max}$ = 304 nm.
(5R,6S)-2-[(2R)-Threonylaminomethyl]-6-[(1R)-1-hydroxyäthyl]-2-penem-3-carbonsäure, UV (Wasser): $\lambda_{max}$ = 305 nm.
(5R,6S)-2-[(2S)-Arginylaminomethyl]-6-[(1R)-1-hydroxyäthyl]-2-penem-3-carbonsäure, UV (Wasser) $\lambda_{max}$ = 304 nm.
(5R,6S)-2-[(2S)-Asparagylaminomethyl]-6-[(1R)-1-hydroxyäthyl]-2-penem-3-carbonsäure, UV (Wasser) $\lambda_{max}$ = 304 nm.

(5R,6S)-2-[(2S)-Glutamylaminomethyl]-6-[(1R)-1-hydroxyäthyl]-2-penem-3-carbonsäure, UV (Wasser) $\lambda_{max}$ = 306 nm.
(5R,6S)-2-[(2S)-Phenylglycylaminomethyl]-6-[(1R)-1-hydroxyäthyl]-2-penem-3-carbonsäure, UV (Wasser) $\lambda_{max}$ = 305 nm.
(5R,6S)-2-[(2S)-4-Hydroxyphenylglycylaminomethyl]-6-[(1R)-1-hydroxyäthyl]-2-penem-3-carbonsäure, UV (Wasser) $\lambda_{max}$ = 307 nm.
(5R,6S)-2-[(2S)-Prolylaminomethyl]-6-[(1R)-1-hydroxyäthyl]-2-penem-3-carbonsäure, UV (Wasser) $\lambda_{max}$ = 304 nm.

Beispiel 8: (5R,6S)-2-Glycylaminomethyl-6-[(1R)-1-hydroxyäthyl]-2-penem-3-carbonsäure

Eine auf 0° gekühlte Lösung von 0,61 g (5R,6S)-2-Aminomethyl-6-[(1R)-1-hydroxyäthyl]-2-penem-3-carbonsäure in 15 ml eines Natriumborat-Puffers (pH 10) wird innerhalb 30 Sekunden und bei heftigem Rühren mit einer Lösung von 0,36 g Glycin-N-carbonsäure-Anhydrid [H. Leuchs, Chem. Ber. 39, 857 (1906)] in 6 ml abs. THF versetzt. Das Gemisch wird, falls erforderlich, durch Zugabe von 50 %iger NaOH zwischen pH 9 und 10 gehalten und bei 0° während 1 Stunde heftig gerührt. Dann wird es mit konz. $H_2SO_4$ auf pH 5 gestellt, 5 Minuten bei diesem pH gerührt und mit Natronlauge neutral gestellt. Nach Einengen auf ein Viertel des Volums wird die Titelverbindung durch Chromatographie an Opti $UPC_{12}$ Silicagel mit Wasser gereinigt.
DC (Reversed Phase Opti $UPC_{12}$, Wasser): $R_f$ = 0.62
UV (Wasser): $\lambda_{max}$ = 304 nm

Beispiel 9: (5R,6S)-2-[(2S)-Alanylaminomethyl]-6-[(1R)-1-hydroxyäthyl]-2-penem-3-carbonsäure

Analog Beispiel 8 werden 0,95 g (5R,6S)-2-Aminomethyl-6-[(1R)-1-hydroxyäthyl]-2-penem-3-carbonsäure mit 0,5 g (2S)-Alanin-N-carbonsäure-Anhydrid in die Titelverbindung überführt
DC (Reversed Phase Opti $UPC_{12}$, Wasser): $R_f$ = 0,56
UV (Wasser): $\lambda_{max}$ = 305 nm

Beispiel 10: (5R,6S)-2-[(2S)-Serylaminomethyl]-6-[(1R)-1-hydroxyäthyl]-2-penem-3-carbonsäure

Analog Beispiel 8 werden 0,36 g (5R,6S)-2-Aminomethyl-6-[(1R)-1-hydroxyäthyl]-2-penem-3-carbonsäure mit 0,35 g O-Trimethylsilyl-(2S)-serin-N-carbonsäure-Anhydrid [R. Hirschmann et al., J. Am. Chem. Soc. 93, 2746 (1971)] in die Titelverbindung überführt.

DC (Reversed Phase Opt: $UPC_{12}$, Wasser): $R_f$ = 0,64

UV (Wasser): $\lambda_{max}$ = 304 nm

Beispiel 11: (5R,6S)-2-[(2R)-Serylaminomethyl]-6-[(1R)-1-hydroxyäthyl]-2-penem-3 -carbonsäure

Analog Beispiel 8 werden 0,7 g (5R,6S)-2-Aminomethyl-6-[(1R)-1-hydroxyäthyl]-2-penem-3-carbonsäure mit 0,7 g O-Trimethylsilyl-(2R)-serin-N-carbonsäure-Anhydrid in die Titelverbindung überführt.

DC (Reversed Phase Opti $UPC_{12}$, Wasser): $R_f$ = 0,6

UV (Wasser): $\lambda_{max}$ = 303 nm

Beispiel 12: (5R,6S)-2-[(2S)-Tyrosylaminomethyl]-6-[(1R)-1-hydroxyäthyl]-2-penem-3 -carbonsäure

Analog Beispiel 8 werden 0,7 g (5R,6S)-2-Aminomethyl-6-[(1R)-1-hydroxyäthyl]-2-penem-3-carbonsäure mit 1 g O-Trimethylsilyl-(2S)-tyrosin-N-carbonsäure-Anhydrid [H.R. Kricheldorf, Chem. Ber. 103, 3353 (1970)] in die Titelverbindung überführt.

DC (Reversed Phase Opti $UPC_{12}$, Wasser): $R_f$ = 0,27

UV (Wasser) $\lambda_{max}$ = 307 nm

Beispiel 13: (5R,6S)-2-[(2R)-Tyrosylaminomethyl]-6-[(1R)-1-hydroxyäthyl]-2-penem-3-carbonsäure

Analog Beispiel 8 werden 0,5 g (5R,6S)-2-Aminomethyl-6-[(1R)-hydroxyäthyl]-2-penem-3-carbonsäure mit 0,7 g O-Trimethylsilyl-(2R)-tyrosin-N-carbonsäure-Anhydrid in die Titelverbindung überführt.

UV (Wasser): $\lambda_{max}$ = 306 nm

Beispiel 14: (5R,6S)-2-[(2S)-Threonylaminomethyl]-6-[(1R)-1-hydroxyäthyl]-2-penem -3-carbonsäure

Analog Beispiel 8 werden 1 g (5R,6S)-2-Aminomethyl-6-[(1R)-1-hydroxyäthyl]-2-penem-3-carbonsäure mit 1,25 g O-Trimethylsilyl-(2S)-threonin-N-carbonsäure-Anhydrid (R. Wies und P. Pfaender, Liebigs Ann. Chem. 1973, 1269) in die Titelverbindung überführt.

DC (Reverse Phase Opti $UPC_{12}$, Wasser): $R_f = 0{,}29$

UV (Wasser): $\lambda_{max} = 304$ nm

Beispiel 15: In analoger Weise, wie in den Beispielen 8 - 14 beschrieben, können die folgenden Verbindungen hergestellt werden:

(5R,6S)-2-[(2R)-Alanylaminomethyl]-6-hydroxymethyl-2-penem-3-carbonsäure, UV (Phosphatpuffer pH 7,4): $\lambda_{max} = 303$ nm.

(5R,6S)-2-[(2S)-Serylaminomethyl]-6-hydroxymethyl-2-penem-3-carbonsäure, UV (Wasser): $\lambda_{max} = 303$ nm.

(5R,6S)-2-[(2R)-Serylaminomethyl]-6-hydroxymethyl-2-penem-3-carbonsäure, UV (Wasser): $\lambda_{max} = 302$ nm.

(5R,6S)-2-[(2S)-Histidylaminomethyl]-6-hydroxymethyl-2-penem-3-carbonsäure, UV (Wasser): $\lambda_{max} = 304$ nm.

(5R,6S)-2-[(2R)-Histidylaminomethyl]-6-hydroxymethyl-2-penem-3-carbonsäure, UV (Wasser): $\lambda_{max} = 303$ nm.

(5R,6S)-2-[(2S)-Histidylaminomethyl]-6-[(1R)-1-hydroxyäthyl]-2-penem-3-carbonsäure, UV (Wasser): $\lambda_{max} = 303{,}5$ nm.

(5R,6S)-2-[(2R)-Histidylaminomethyl]-6-[(1R)-1-hydroxyäthyl]-2-penem-3-carbonsäure, UV (Wasser): $\lambda_{max} = 304$ nm.

(5R,6S)-2-[(2R)-Threonylaminomethyl]-6-[(1R)-1-hydroxyäthyl]-2-penem-3-carbonsäure UV (Wasser): $\lambda_{max} = 305$ nm.

(5R,6S)-2-[(2S)-Arginylaminomethyl]-6-[(1R)-1-hydroxyäthyl]-2-penem-3-carbonsäure, UV (Wasser) $\lambda_{max} = 304$ nm.

(5R,6S)-2-[(2S)-Asparagylaminomethyl]-6-[(1R)-1-hydroxyäthyl]-2-penem-3-carbonsäure, UV (Wasser) $\lambda_{max} = 304$ nm.

(5R,6S)-2-[(2S)-Glutamylaminomethyl]-6-[(1R)-1-hydroxyäthyl]-2-penem-3-carbonsäure, UV (Wasser) $\lambda_{max} = 306$ nm.

(5R,6S)-2-[(2S)-Phenylglycylaminomethyl]-6-[(1R)-1-hydroxyäthyl]-2-penem-3-carbonsäure, UV (Wasser) $\lambda_{max} = 305$ nm.

(5R,6S)-2-[(2S)-4-Hydroxyphenylglycylaminomethyl]-6-[(1R)-1-hydroxyäthyl]-2-penem-3-carbonsäure, UV (Wasser) $\lambda_{max}$ = 307 nm.
(5R,6S)-2-[(2S)-Prolylaminomethyl]-6-[(1R)-1-hydroxyäthyl]-2-penem-3-carbonsäure, UV (Wasser) $\lambda_{max}$ = 304 nm.

Beispiel 16: Trockenampullen oder Vials, enthaltend 0,5 g (5R,6S)-2-Glycylaminomethyl-6-[(1R)-1-hydroxyäthyl]-2-penem-3-carbonsäure als Wirksubstanz, werden wie folgt hergestellt:

Zusammensetzung (für 1 Ampulle oder Vial):

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des oben genannten Wirkstoffs kann auch dieselbe Menge eines anderen Wirkstoffs der vorangehenden Beispiele, wie z.B. die (5R,6S)-2-[(2S)-Alanylaminomethyl]-6-[(1R)-1-hydroxyäthyl]-2-penem-3-carbonsäure, verwendet werden.

Beispiel 17: Kapseln, enthaltend 0,250 g (5R,6S)-2-Glycylaminomethyl-6-[(1R)-1-hydroxyäthyl]-2-penem-3-carbonsäure als Wirkstoff, werden wie folgt hergestellt:

Zusammensetzung (für 100 000 Kapseln)

| | |
|---|---|
| Wirkstoff | 25 000 g |
| Weizenstärke | 2 500 g |
| Magnesiumstearat | 1 000 g |

Der Wirkstoff, die Weizenstärke und das Magnesiumstearat werden gut miteinander vermischt und in Kapseln abgefüllt.

Anstelle des oben genannten Wirkstoffs kann auch dieselbe Menge eines anderen Wirkstoffs der vorangehenden Beispiele, wie z.B. die (5R,6S)-2-[(2S)-Alanylaminomethyl]-6-[(1R)-1-hydroxyäthyl]-2-penem-3-carbonsäure, verwendet werden.

Patentansprüche für die Vertragsstaaten: DE, GB, FR, CH, LI, IT, NL, BE, SE, LU

1. Verbindungen der Formel

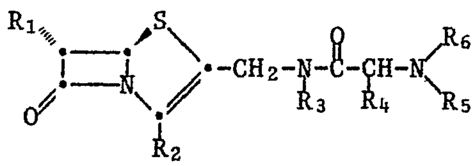

(I),

worin $R_1$ durch Hydroxy oder geschütztes Hydroxy substituiertes Niederalkyl ist, $R_2$ Carboxyl oder funktionell abgewandeltes Carboxyl ist, $R_3$ Wasserstoff oder Niederalkyl darstellt, $R_4$ für Wasserstoff oder für einen über ein Kohlenstoffatom gebundenen organischen Rest steht, $R_5$ Wasserstoff oder Niederalkyl bedeutet, oder zusammen mit $R_4$ gegebenenfalls durch Hydroxy oder Oxo substituiertes Niederalkylen bedeutet, und $R_6$ Wasserstoff, Niederalkyl oder Acyl ist, optische Isomere von Verbindungen der Formel I, Mischungen dieser optischen Isomere und Salze von solchen Verbindungen der Formel I, die eine salzbildende Gruppe aufweisen.

2. Verbindungen der Formel I gemäss Anspruch 1, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl $R_2'$ bedeutet; $R_3$ Wasserstoff oder Niederalkyl ist; $R_4$ Wasserstoff oder Niederalkyl, Niederalkenyl, Cycloalkyl, Cycloalkenyl, Cycloalkadienyl, Phenyl, Naphthyl, Phenylniederalkyl, einen über ein Ringkohlenstoffatom gebundenen monocyclischen 5- oder 6-gliedrigen gegebenenfalls partiell gesättigten Heteroarylrest mit 1 - 4 Ringstickstoffatomen und gegebenenfalls einem zusätzlichen Ringheteroatom, ausgewählt aus der Gruppe Sauerstoff und Schwefel, oder einen entsprechenden Dihydro- oder Tetrahydrorest, oder ein gegebenenfalls partiell gesättigtes

Benzo-, Dibenzo-, Pyrido- oder Pyrimido-Derivat eines solchen 5- oder 6-gliedrigen Restes, oder einen durch einen der genannten, über ein Ringkohlenstoffatom gebundenen, gegebenenfalls partiell gesättigten Heteroarylreste oder durch einen über ein Ringstickstoffatom gebundenen monocyclischen, 5- oder 6-gliedrigen gegebenenfalls partiell gesättigten Heterocyclylrest mit 1-4 Ringstickoffatomen substituierter Niederalkylrest darstellt, welche Reste $R_4$ unsubstituiert oder durch Hydroxy, Niederalkoxy, Phenylniederalkoxy, Niederalkanoyloxy, Halogen, Mercapto, Niederalkylthio, Phenylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl, Niederalkylen, gegebenenfalls niederalkyliertes Azaniederalkylen, Oxaniederalkylen, Dioxaniederalkylen, Thianiederalkylen, Amino, Niederalkylamino, Diniederalkylamino, Niederalkylenamino, Guanidino, Carbamoylamino, Niederalkanoylamino, gegebenenfalls durch Amino und/oder Carboxy substituiertes Niederalkoxycarbonylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Cycloalkyl, Nitro, Oxo und/oder Oxido substituiert sind; $R_5$ Wasserstoff, Niederalkyl oder zusammen mit $R_4$ gegebenenfalls durch Hydroxy oder Oxo substituiertes Niederalkylen ist; und $R_6$ Wasserstoff, Niederalkyl, Niederalkanoyl, durch Hydroxy, Niederalkoxy, Amino, Halogen, Carboxy und/oder Cyan substituiertes Niederalkanoyl; Niederalkenoyl, Cycloalkanoyl, Benzoyl, durch Amino, Niederalkylamino, Diniederalkylamino, Niederalkanoylamino, Carboxy, Halogen, Nitro, Cyano und/oder Niederalkyl substituiertes Benzoyl, gegebenenfalls durch Amino oder Hydroxy substituiertes Phenylniederalkanoyl, Heterocyclylcarbonyl, worin Heterocyclyl einer der bei der Definition von $R_4$ angegebenen gegebenenfalls partiell gesättigten, gegebenenfalls substituierten 5- oder 6-gliedrigen monocyclischen Heteroarylreste oder ein gegebenenfalls durch Oxo, Sulfo, Niederalkoxysulfonyl, Sulfamoyl, Niederalkyl und/oder Hydroxy

substituierter gesättigter 5- oder 6-gliedriger Diazacycloalkan-Rest bedeutet; Niederalkoxycarbonyl, durch Carboxy und Amino substituiertes Niederalkoxycarbonyl, Benzyloxycarbonyl, Carbamoyl, Anilinocarbonyl, N-mono- oder N,N-diniederalkyliertes Carbamoyl, Niederalkyl-thiocarbamoyl, Sulfo, Niederalkansulfonyl, durch Halogen substituiertes Niederalkansulfonyl, Benzolsulfonyl, durch Amino, Halogen, Niederalkyl oder Nitro substituiertes Benzolsulfonyl, Sulfamoyl oder eine Aminoschutzgruppe ist, optische Isomere von Verbindungen der Formel I, welche insbesondere im Rest $R_1$ und/oder am Methin-Kohlenstoffatom $-CH(R_4)(NR_5R_6)$ Chiralitätszentren besitzen, Mischungen dieser optischen Isomere und Salze von solchen Verbindungen der Formel I, die eine salzbildende Gruppe aufweisen.

3. Verbindungen der Formel I gemäss Anspruch 1, worin $R_1$ in α-Stellung durch Hydroxy oder geschütztes Hydroxy substituiertes Niederalkyl ist; $R_2$ Carboxyl, geschütztes Carboxyl oder eine unter physiologischen Bedingungen spaltbare veresterte Carboxylgruppe bedeutet; $R_3$ Wasserstoff oder Niederalkyl ist; $R_4$ Wasserstoff, Niederalkyl, durch Hydroxy, Niederalkoxy, Halogen, Mercapto, Niederalkylthio, Amino, Niederalkylamino, Diniederalkylamino, Niederalkanoylamino, Guanidino, Carbamoylamino, Carboxy, Niederalkoxycarbonyl, Carbamoyl, Sulfo und/oder Oxo substituiertes Niederalkyl, Cycloalkenyl mit 5 oder 6 Kohlenstoffatomen, Cycloalkadienyl mit 6 Kohlenstoffatomen, Phenyl, durch Hydroxy, Niederalkoxy, Amino, Niederalkylamino, Diniederalkylamino, Aminoniederalkyl, durch Amino und Carboxy substituiertes Niederalkoxycarbonylamino oder durch Carboxy und Amino substituiertes Niederalkoxycarbonylaminoniederalkyl substituiertes Phenyl; gegebenenfalls durch Niederalkyl, Nitro, Amino, Hydroxy, Niederalkoxy, Carboxy und/oder Halogen substituiertes Phenylniederalkyl; über ein Ringkohlenstoffatom gebundenes, gegebenenfalls durch Niederalkyl, Aminoniederalkyl oder Amino substituiertes Imidazolyl, Pyrazolyl, gegebenenfalls durch Amino substituiertes Triazolyl, gegebenenfalls durch Niederalkyl, Carboxyniederalkyl, Sulfoniederalkyl oder Diniederalkylaminoniederalkyl substituiertes Tetrazolyl, gegebenenfalls durch Niederalkyl oder Amino substituiertes Thiazolyl, Isothiazolyl, gegebenenfalls durch Niederalkyl, Hydroxy, Amino oder Niederalkylamino substituiertes Thiadiazolyl, gegebenenfalls durch Amino oder Niederalkyl substituiertes Oxazolyl, gegebenenfalls durch Niederalkyl oder Amino substituiertes Oxadiazolyl, gegebenenfalls durch Halogen, Niederalkyl oder Aminoniederalkyl substituiertes Furyl, gegebenenfalls durch Halogen, Niederalkyl oder Aminoniederalkyl substituiertes Thienyl, gegebenenfalls durch Halogen, Amino und/oder Oxido substituiertes Pyridyl; gegebenenfalls durch Niederalkyl substituiertes Imidazol-4-ylmethyl, gegebenenfalls durch Oxo und gegebenenfalls zusätzlich durch Halogen substituiertes Pyridyl- oder Dihydropyridylmethyl, Furylmethyl, Thienylmethyl, gegebenenfalls durch Amino substituiertes Oxazolyl-, Thiazolyl- oder Thiadiazolylmethyl, oder Indol-3-ylmethyl darstellt; $R_5$ Wasserstoff, Niederalkyl oder zusammen mit $R_4$ gegebenenfalls durch Hydroxy oder Oxo substituiertes Niederalkylen ist; $R_6$ Wasserstoff, Niederalkyl, Niederalkanoyl, durch Hydroxy, Carboxy und/oder Amino substituiertes Niederalkanoyl, Phenylniederalkanoyl, worin Niederalkanoyl in α-Stellung zum Phenylrest durch Hydroxy oder Amino substituiert ist, gegebenenfalls durch Hydroxy und/oder Niederalkyl substituiertes Pyridylcarbonyl, Furoyl, Thenoyl, durch Carboxy substituiertes Imidazolylcarbonyl, durch Oxo, Sulfamoyl und/oder Niederalkyl substituiertes Tetrahydroimidazolylcarbonyl, durch Hydroxy und/oder Niederalkyl substituiertes Piperazinylcarbonyl, durch Hydroxy substituiertes Thiadiazolylcarbonyl, Niederalkoxycarbonyl, durch Amino und Carboxy substituiertes Niederalkoxycarbonyl, Benzyloxycarbonyl, Carbamoyl, Niederalkylcarbamoyl, Niederalkansulfonyl oder Sulfamoyl ist; optische Isomere von Verbindungen der Formel I, welche insbesondere im Rest $R_1$ und/oder am Methin-Kohlenstoffatom $-CH(R_4)(NR_5R_6)$ Chiralitätszentren besitzen, Mischungen dieser optischen Isomere und Salze von solchen Verbindungen der Formel I, die eine salzbildende Gruppe aufweisen.

4. Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl ist; $R_3$ Wasserstoff bedeutet; $R_4$ Wasserstoff, Niederalkyl, durch Hydroxy, Mercapto, Niederalkylthio, Amino, Guanidino, Carbamoylamino, Carboxy oder Carbamoyl substituiertes Niederalkyl, Cyclohexadienyl, gegebenenfalls durch Hydroxy oder Amino substituiertes Phenyl, gegebenenfalls durch Hydroxy substituiertes Phenylmethyl, über ein Kohlenstoffatom gebundenes Aminooxazolyl, Aminothiazolyl, Aminothiadiazolyl, Hydroxythiadiazolyl, Pyridyl, Aminoimidazolyl, Imidazol-4-ylmethyl, Aminothiazol-4-ylmethyl oder Indol-3-ylmethyl bedeutet; $R_5$ Wasserstoff oder zusammen mit $R_4$ 1,3-Propylen oder 2-Hydroxy-1,3-propylen bedeutet; und $R_6$ Wasserstoff, Methyl oder Niederalkanoyl ist, optische Isomere von Verbindungen der Formel I, worin $R_1$ 1-Hydroxyäthyl ist und/oder welche am Methin-Kohlenstoffatom $-CH(R_4)(NR_5R_6)$ ein Chiralitätszentrum besitzen, Mischungen dieser optischen Isomere und Salze von solchen Verbindungen der Formel I, die eine salzbildende Gruppe aufweisen.

5. Verbindungen der Formel I gemäss Anspruch 1, worin $R_1$ Hydroxymethyl oder 1-Hydroxyäthyl bedeutet, $R_2$ Carboxyl oder eine unter physiologischen Bedingungen spaltbare veresterte Carboxylgruppe ist und der Rest $-C(=O)-(CH-R_4)-N(R_5,R_6)$ für den Acylrest einer genetisch enkodierten Aminosäure, für α-Aminobutyryl, Phenylglycyl, 4-Hydroxyphenylglycyl, Citrullyl, Ornithyl, 3-Hydroxyprolyl, Pyrrolidin-5-on-2-carbonyl oder für ein N-Niederalkyl-Derivat eines solchen Acylrestes steht, optische Isomere von Verbindungen der Formel (I), worin $R_1$ 1-Hydroxyäthyl ist und/oder welche am Methin-Kohlenstoffatom $-CH(R_4)(NR_5R_6)$ ein Chiralitätszentrum besitzen, Mischungen solcher optischen Isomere, und Salze von solchen Verbindungen der Formel (I), die eine salzbildende Gruppe aufweisen.

6. Verbindungen der Formel I gemäss Anspruch 1, worin $R_1$ Hydroxymethyl oder 1-Hydroxyäthyl ist, $R_2$ Carboxyl bedeutet, $R_3$, $R_5$ und $R_6$ Wasserstoff sind und $R_4$ Wasserstoff, Methyl, Hydroxymethyl oder Imidazol-4-ylmethyl darstellt, optische Isomere von Verbindungen der Formel I, worin $R_1$ 1-Hydroxyäthyl bedeutet und/oder worin $R_4$ von Wasserstoff verschieden ist, Mischungen dieser optischen Isomere und Salze von solchen Verbindungen der Formel I, die eine salzbildende Gruppe aufweisen.

7. Pharmazeutisch annehmbare Salze von Verbindungen der Formel I gemäss Anspruch 1.

8. Unter physiologischen Bedingungen spaltbare Ester von Verbindungen der Formel I gemäss Anspruch 1.

9. Verbindungen der Formel I gemäss Anspruch 1, worin $R_1$ (1R)-1-Hydroxyäthyl ist und $R_2$, $R_3$, $R_4$, $R_5$ und $R_6$ die in Anspruch 1 angegebenen Bedeutungen haben, und Salze von solchen Verbindungen der Formel I.

10. (5R,6S)-2-Glycylaminomethyl-6-hydroxymethyl-2-penem-3-carbonsäure, pharmazeutisch annehmbare Salze davon und unter physiologischen Bedingungen spaltbare Ester davon gemäss Anspruch 1.

11. (5R,6S)-2-[(2S)-Alanylaminomethyl]-6-hydroxymethyl-2-penem-3-carbonsäure, pharmazeutisch annehmbare Salze davon und unter physiologischen Bedingungen spaltbare Ester davon gemäss Anspruch 1.

12. (5R,6S)-2-[(2S)-Serylaminomethyl]-6-hydroxymethyl-2-penem-3-carbonsäure, pharmazeutisch annehmbare Salze davon und unter physiologischen Bedingungen spaltbare Ester davon gemäss Anspruch 1.

13. (5R,6S)-2-Glycylaminomethyl-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

14. (5R,6S)-2-[(2S)-Alanylaminomethyl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

15. (5R,6S)-2-[(2S)-Tyrosylaminomethyl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

16. (5R,6S)-2-[(2S)-Threonylaminomethyl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

17. (5R,6S)-2-[(2S)-Serylaminomethyl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

18. (5R,6S)-2-[(2R)-Serylaminomethyl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

19. Pharmazeutisches Präparat enthaltend eine Verbindung der Formel I gemäss Anspruch 1, ein pharmazeutisch annehmbares Salz oder einen unter physiologischen Bedingungen spaltbaren Ester einer solchen Verbindung.

20. Verwendung von Verbindungen der Formel I gemäss Anspruch 1, von pharmazeutisch annehmbaren Salzen und von unter physiologischen Bedingungen spaltbaren Estern solcher Verbindungen zur Herstellung von pharmazeutischen Präparaten.

21. Eine Verbindung der Formel I gemäss Anspruch 1, pharmazeutisch annehmbare Salze und unter physiologischen Bedingungen spaltbare Ester von solchen Verbindungen als antibiotische Mittel.

22. Eine Verbindung der Formel I gemäss Anspruch 1, pharmazeutisch annehmbare Salze und unter physiologischen Bedingungen spaltbare Ester von solchen Verbindungen zur Anwendung in einem Verfahren zur therapeutischen Behandlung des menschlichen oder tierischen Körpers.

23. Verfahren zur Herstellung von Verbindungen der Formel I gemäss Anspruch 1, dadurch gekennzeichnet, dass man

a) in einer Verbindung der Formel

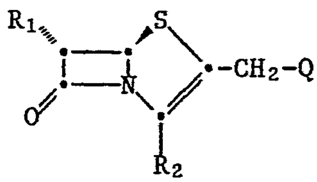

(II),

worin $R_1$ und $R_2$ die unter Formel (I) angegebene Bedeutung haben, und Q eine in den Rest

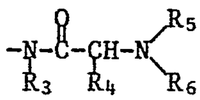

(III)

überführbare Gruppe ist, die Gruppe Q in den Rest

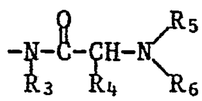

(III)

überführt, oder

b) eine Ylid-Verbindung der Formel

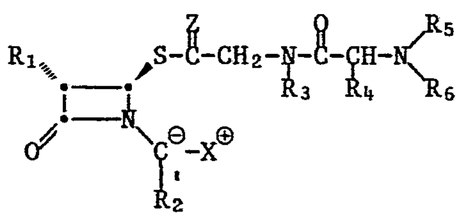

(V) ,

worin $R_1$, $R_3$, $R_4$, $R_5$ und $R_6$ die unter Formel I angegebenen Bedeutungen haben, $R_2$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

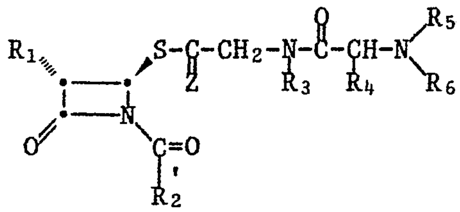

(VI) ,

worin $R_1$, $R_3$, $R_4$, $R_5$ und $R_6$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, oder

d) in einer Verbindung der Formel

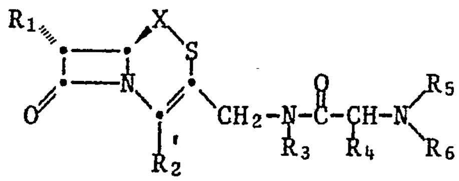

(VII) ,

worin $R_1$, $R_3$, $R_4$, $R_5$ und $R_6$ die unter Formel I angegebenen Bedeutungen haben, $R_2'$ eine geschützte Carboxylgruppe darstellt und X für die Gruppe -S- oder für die Gruppe $-SO_2-$ steht, den Rest X abspaltet, und wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt und/oder, wenn erwünscht, in einer erhältlichen Verbindung der Formel I einen Rest $R_3$ und/oder $R_4$ und/oder $R_5$ und/oder $R_6$ in einen anderen Rest $R_3$ und/oder $R_4$ und/oder $R_5$ und/oder $R_6$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

24. Die nach dem Verfahren gemäss Anspruch 23 erhältlichen Verbindungen.

FO 7.4/UL/cs*

Patentansprüche für den Vertragsstaat: AT

1. Verfahren zur Herstellung von Verbindungen der Formel

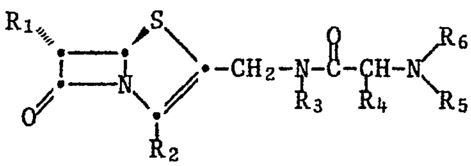

(I),

worin $R_1$ durch Hydroxy oder geschütztes Hydroxy substituiertes Niederalkyl ist, $R_2$ Carboxyl oder funktionell abgewandeltes Carboxyl ist, $R_3$ Wasserstoff oder Niederalkyl darstellt, $R_4$ für Wasserstoff oder für einen über ein Kohlenstoffatom gebundenen organischen Rest steht, $R_5$ Wasserstoff oder Niederalkyl bedeutet, oder zusammen mit $R_4$ gegebenenfalls durch Hydroxy oder Oxo substituiertes Niederalkylen bedeutet, und $R_6$ Wasserstoff, Niederalkyl oder Acyl ist, optischen Isomeren von Verbindungen der Formel I, Mischungen dieser optischen Isomere und Salzen von solchen Verbindungen der Formel I, die eine salzbildende Gruppe aufweisen, dadurch gekennzeichnet, dass man

a) in einer Verbindung der Formel

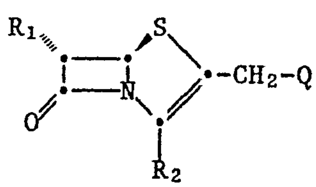

(II),

worin $R_1$ und $R_2$ die unter Formel (I) angegebene Bedeutung haben, und Q eine in den Rest

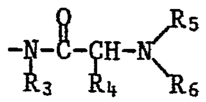

(III)

überführbare Gruppe ist, die Gruppe Q in den Rest

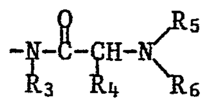

(III)

überführt, oder

b) eine Ylid-Verbindung der Formel

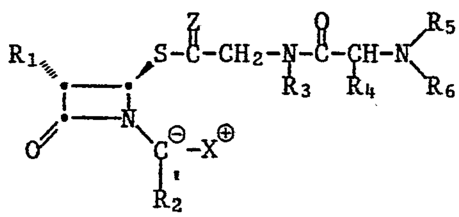

(V) ,

worin $R_1$, $R_3$, $R_4$, $R_5$ und $R_6$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

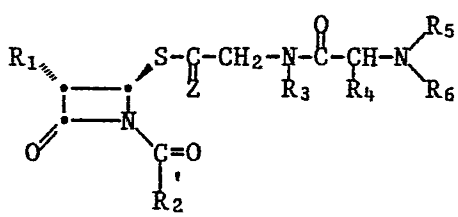

(VI) ,

worin $R_1$, $R_3$, $R_4$, $R_5$ und $R_6$ die unter Formel I angegebenen Bedeutungen haben, Z Sauerstoff oder Schwefel ist und $R_2^1$ eine geschützte Carboxylgruppe ist, mit einer organischen Verbindung des dreiwertigen Phosphors behandelt, oder

d) in einer Verbindung der Formel

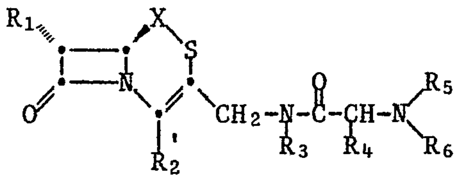

(VII) ,

worin $R_1$, $R_3$, $R_4$, $R_5$ und $R_6$ die unter Formel I angegebenen Bedeutungen haben, $R_2'$ eine geschützte Carboxylgruppe darstellt und X für die Gruppe -S- oder für die Gruppe $-SO_2-$ steht, den Rest X abspaltet, und wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt und/oder, wenn erwünscht, in einer erhältlichen Verbindung der Formel I einen Rest $R_3$ und/oder $R_4$ und/oder $R_5$ und/oder $R_6$ in einen anderen Rest $R_3$ und/oder $R_4$ und/oder $R_5$ und/oder $R_6$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen der Formel I in die einzelnen Isomere auftrennt.

2. Verfahren gemäss Anspruch 1 zur Herstellung von Verbindungen der Formel I, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl $R_2'$ bedeutet; $R_3$ Wasserstoff oder Niederalkyl ist; $R_4$ Wasserstoff oder Niederalkyl, Niederalkenyl, Cycloalkyl, Cycloalkenyl, Cycloalkadienyl, Phenyl, Naphthyl, Phenylniederalkyl, einen über ein Ringkohlenstoffatom gebundenen monocyclischen 5- oder 6-gliedrigen gegebenenfalls partiell gesättigten Heteroarylrest mit 1 - 4 Ringstickstoffatomen und gegebenenfalls einem zusätzlichen

Ringheteroatom, ausgewählt aus der Gruppe Sauerstoff und Schwefel, oder einen entsprechenden Dihydro-oder Tetrahydrorest, oder ein gegebenenfalls partiell gesättigtes Benzo-, Dibenzo-, Pyrido- oder Pyrimido-Derivat eines solchen 5- oder 6-gliedrigen Restes, oder einen durch einen der genannten, über ein Ringkohlenstoffatom gebundenen, gegebenenfalls partiell gesättigten Heteroarylreste oder durch einen über ein Ringstickstoffatom gebundenen monocyclischen, 5- oder 6-gliedrigen gegebenenfalls partiell gesättigten Heterocyclylrest mit 1 - 4 Ringstickoffatomen substituierter Niederalkylrest darstellt, welche Reste $R_4$ unsubstituiert oder durch Hydroxy, Niederalkoxy, Phenylniederalkoxy, Niederalkanoyloxy, Halogen, Mercapto, Niederalkylthio, Phenylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl, Niederalkylen, gegebenenfalls niederalkyliertes Azaniederalkylen, Oxaniederalkylen, Dioxaniederalkylen, Thianiederalkylen, Amino, Niederalkylamino, Diniederalkylamino, Niederalkylenamino, Guanidino, Carbamoylamino, Niederalkanoylamino, gegebenenfalls durch Amino und/oder Carboxy substituiertes Niederalkoxycarbonylamino, Carboxyl, Niederalkoxycarbonyl, Carbamoyl, N-mono-oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Cycloalkyl, Nitro, Oxo und/oder Oxido substituiert sind; $R_5$ Wasserstoff, Niederalkyl oder zusammen mit $R_4$ gegebenenfalls durch Hydroxy oder Oxo substituiertes Niederalkylen ist; und $R_6$ Wasserstoff, Niederalkyl, Niederalkanoyl, durch Hydroxy, Niederalkoxy, Amino, Halogen, Carboxy und/oder Cyan substituiertes Niederalkanoyl; Niederalkenoyl, Cycloalkanoyl, Benzoyl, durch Amino, Niederalkylamino, Diniederalkylamino, Niederalkanoylamino, Carboxy, Halogen, Nitro, Cyano und/oder Niederalkyl substituiertes Benzoyl, gegebenenfalls durch Amino oder Hydroxy substituiertes Phenylniederalkanoyl, Heterocyclylcarbonyl, worin Heterocyclyl einer der bei der Definition von $R_4$ angegebenen gegebenenfalls partiell gesättigten,

gegebenenfalls substituierten 5- oder 6-gliedrigen monocyclischen Heteroarylreste oder ein gegebenenfalls durch Oxo, Sulfo, Niederalkoxysulfonyl, Sulfamoyl, Niederalkyl und/oder Hydroxy substituierter gesättigter 5- oder 6-gliedriger Diazacycloalkan-Rest bedeutet; Niederalkoxycarbonyl, durch Carboxy und Amino substituiertes Niederalkoxycarbonyl, Benzyloxycarbonyl, Carbamoyl, Anilinocarbonyl, N-mono- oder N,N-diniederalkyliertes Carbamoyl, Niederalkyl-thiocarbamoyl, Sulfo, Niederalkansulfonyl, durch Halogen substituiertes Niederalkansulfonyl, Benzolsulfonyl, durch Amino, Halogen, Niederalkyl oder Nitro substituiertes Benzolsulfonyl, Sulfamoyl oder eine Aminoschutzgruppe ist, optischen Isomeren von Verbindungen der Formel I, welche insbesondere im Rest $R_1$ und/oder am Methin-Kohlenstoffatom $-CH(R_4)(NR_5R_6)$ Chiralitätszentren besitzen, Mischungen dieser optischen Isomere und Salzen von solchen Verbindungen der Formel I, die eine salzbildende Gruppe aufweisen.

3. Verfahren gemäss Anspruch 1 zur Herstellung von Verbindungen der Formel I, worin $R_1$ in $\alpha$-Stellung durch Hydroxy oder geschütztes Hydroxy substituiertes Niederalkyl ist; $R_2$ Carboxyl, geschütztes Carboxyl oder eine unter physiologischen Bedingungen spaltbare veresterte Carboxylgruppe bedeutet; $R_3$ Wasserstoff oder Niederalkyl ist; $R_4$ Wasserstoff, Niederalkyl, durch Hydroxy, Niederalkoxy, Halogen, Mercapto, Niederalkylthio, Amino, Niederalkylamino, Diniederalkylamino, Niederalkanoylamino, Guanidino, Carbamoylamino, Carboxy, Niederalkoxycarbonyl, Carbamoyl, Sulfo und/oder Oxo substituiertes Niederalkyl, Cycloalkenyl mit 5 oder 6 Kohlenstoffatomen, Cycloalkadienyl mit 6 Kohlenstoffatomen, Phenyl, durch Hydroxy, Niederalkoxy, Amino, Niederalkylamino, Diniederalkylamino, Aminoniederalkyl, durch Amino und Carboxy substituiertes Niederalkoxycarbonylamino oder durch Carboxy und Amino substituiertes Niederalkoxycarbonylaminoniederalkyl substituiertes Phenyl; gegebenenfalls durch Niederalkyl, Nitro, Amino, Hydroxy, Niederalkoxy, Carboxy und/oder Halogen substituiertes Phenylniederalkyl; über ein Ringkohlenstoffatom gebundenes, gegebenenfalls durch Niederalkyl, Aminoniederalkyl oder Amino substituiertes Imidazolyl, Pyrazolyl, gegebenenfalls durch Amino substituiertes Triazolyl, gegebenenfalls

durch Niederalkyl, Carboxyniederalkyl, Sulfoniederalkyl oder Diniederalkylaminoniederalkyl substituiertes Tetrazolyl, gegebenenfalls durch Niederalkyl oder Amino substituiertes Thiazolyl, Isothiazolyl, gegebenenfalls durch Niederalkyl, Hydroxy, Amino oder Niederalkylamino substituiertes Thiadiazolyl, gegebenenfalls durch Amino oder Niederalkyl substituiertes Oxazolyl, gegebenenfalls durch Niederalkyl oder Amino substituiertes Oxadiazolyl, gegebenenfalls durch Halogen, Niederalkyl oder Aminoniederalkyl substituiertes Furyl, gegebenenfalls durch Halogen, Niederalkyl oder Aminoniederalkyl substituiertes Thienyl, gegebenenfalls durch Halogen, Amino und/oder Oxido substituiertes Pyridyl; gegebenenfalls durch Niederalkyl substituiertes Imidazol-4-ylmethyl, gegebenenfalls durch Oxo und gegebenenfalls zusätzlich durch Halogen substituiertes Pyridyl- oder Dihydropyridylmethyl, Furylmethyl, Thienylmethyl, gegebenenfalls durch Amino substituiertes Oxazolyl-, Thiazolyl- oder Thiadiazolylmethyl, oder Indol-3-ylmethyl darstellt; $R_5$ Wasserstoff, Niederalkyl oder zusammen mit $R_4$ gegebenenfalls durch Hydroxy oder Oxo substituiertes Niederalkylen ist; $R_6$ Wasserstoff, Niederalkyl, Niederalkanoyl, durch Hydroxy, Carboxy und/oder Amino substituiertes Niederalkanoyl, Phenylniederalkanoyl, worin Niederalkanoyl in α-Stellung zum Phenylrest durch Hydroxy oder Amino substituiert ist, gegebenenfalls durch Hydroxy und/oder Niederalkyl substituiertes Pyridylcarbonyl, Furoyl, Thenoyl, durch Carboxy substituiertes Imidazolylcarbonyl, durch Oxo, Sulfamoyl und/oder Niederalkyl substituiertes Tetrahydroimidazolylcarbonyl, durch Hydroxy und/oder Niederalkyl substituiertes Piperazinylcarbonyl, durch Hydroxy substituiertes Thiadiazolylcarbonyl, Niederalkoxycarbonyl, durch Amino und Carboxy substituiertes Niederalkoxycarbonyl, Benzyloxycarbonyl, Carbamoyl, Niederalkylcarbamoyl, Niederalkansulfonyl oder Sulfamoyl ist; optischen Isomeren von Verbindungen der Formel I, welche insbesondere im Rest $R_1$ und/oder am Methin-Kohlenstoffatom $-CH(R_4)(NR_5R_6)$ Chiralitätszentren besitzen, Mischungen dieser optischen Isomere und Salzen von solchen Verbindungen der Formel I, die eine salzbildende Gruppe aufweisen.

4. Verfahren gemäss Anspruch 1 zur Herstellung von Verbindungen der Formel I, worin $R_1$ Hydroxymethyl oder 1-Hydroxyäthyl ist, $R_2$ Carboxyl oder unter physiologischen Bedingungen spaltbares verestertes Carboxyl ist; $R_3$ Wasserstoff bedeutet; $R_4$ Wasserstoff, Niederalkyl, durch Hydroxy, Mercapto, Niederalkylthio, Amino, Guanidino, Carbamoylamino, Carboxy oder Carbamoyl substituiertes Niederalkyl, Cyclohexadienyl, gegebenenfalls durch Hydroxy oder Amino substituiertes Phenyl, gegebenenfalls durch Hydroxy substituiertes Phenylmethyl, über ein Kohlenstoffatom gebundenes Aminooxazolyl, Aminothiazolyl, Aminothiadiazolyl, Hydroxythiadiazolyl, Pyridyl, Aminoimidazolyl, Imidazol-4-ylmethyl, Aminothiazol-4-ylmethyl oder Indol-3-ylmethyl bedeutet; $R_5$ Wasserstoff oder zusammen mit $R_4$ 1,3-Propylen oder 2-Hydroxy-1,3-propylen bedeutet; und $R_6$ Wasserstoff, Methyl oder Niederalkanoyl ist, optischen Isomeren von Verbindungen der Formel I, worin $R_1$ 1-Hydroxyäthyl ist und/oder welche am Methin-Kohlenstoffatom $-CH(R_4)(NR_5R_6)$ ein Chiralitätszentrum besitzen, Mischungen dieser optischen Isomere und Salzen von solchen Verbindungen der Formel I, die eine salzbildende Gruppe aufweisen.

5. Verfahren gemäss Anspruch 1 zur Herstellung von Verbindungen der Formel I, worin $R_1$ Hydroxymethyl oder 1-Hydroxyäthyl bedeutet, $R_2$ Carboxyl oder eine unter physiologischen Bedingungen spaltbare veresterte Carboxylgruppe ist und der Rest $-C(=O)-(CH-R_4)-N(R_5,R_6)$ für den Acylrest einer genetisch enkodierten Aminosäure, für α-Aminobutyryl, Phenylglycyl, 4-Hydroxyphenylglycyl, Citrullyl, Ornithyl, 3-Hydroxyprolyl, Pyrrolidin-5-on-2-carbonyl oder für ein N-Niederalkyl-Derivat eines solchen Acylrestes steht, optischen Isomeren von Verbindungen der Formel (I), worin $R_1$ 1-Hydroxyäthyl ist und/oder welche am Methin-Kohlenstoffatom $-CH(R_4)(NR_5R_6)$ ein Chiralitätszentrum besitzen, Mischungen solcher optischen Isomere, und Salzen von solchen Verbindungen der Formel (I), die eine salzbildende Gruppe aufweisen.

6. Verfahren gemäss Anspruch 1 zur Herstellung von Verbindungen der Formel I, worin $R_1$ Hydroxymethyl oder 1-Hydroxyäthyl ist, $R_2$ Carboxyl bedeutet, $R_3$, $R_5$ und $R_6$ Wasserstoff sind und $R_4$ Wasserstoff, Methyl, Hydroxymethyl oder Imidazol-4-ylmethyl darstellt, optischen Isomeren von Verbindungen der Formel I, worin $R_1$ 1-Hydroxyäthyl bedeutet und/oder worin $R_4$ von Wasserstoff verschieden ist, Mischungen dieser optischen Isomere und Salzen von solchen Verbindungen der Formel I, die eine salzbildende Gruppe aufweisen.

7. Verfahren zur Herstellung von pharmazeutisch annehmbaren Salzen von Verbindungen der Formel I gemäss Anspruch 1.

8. Verfahren zur Herstellung von unter physiologischen Bedingungen spaltbaren Estern von Verbindungen der Formel I gemäss Anspruch 1.

9. Verfahren gemäss Anspruch 1 zur Herstellung von Verbindungen der Formel I, worin $R_1$ (1R)-1-Hydroxyäthyl ist und $R_2$, $R_3$, $R_4$, $R_5$ und $R_6$ die in Anspruch 1 angegebenen Bedeutungen haben, und Salzen von solchen Verbindungen.

10. Verfahren zur Herstellung von (5R,6S)-2-Glycylaminomethyl-6-hydroxymethyl-2-penem-3-carbonsäure, pharmazeutisch annehmbaren Salzen davon und unter physiologischen Bedingungen spaltbaren Estern davon gemäss Anspruch 1.

11. Verfahren zur Herstellung von (5R,6S)-2-[(2S)-Alanylaminomethyl]-6-hydroxymethyl-2-penem-3-carbonsäure, pharmazeutisch annehmbaren Salzen davon und unter physiologischen Bedingungen spaltbaren Estern davon gemäss Anspruch 1.

12. Verfahren zur Herstellung von (5R,6S)-2-[(2S)-Serylaminomethyl]-6-hydroxymethyl-2-penem-3-carbonsäure, pharmazeutisch annehmbaren Salzen davon und unter physiologischen Bedingungen spaltbaren Estern davon gemäss Anspruch 1.

13. Verfahren zur Herstellung von (5R,6S)-2-Glycylaminomethyl-6-[(1R)-1-hydroxyäthyl]-2-penem-3-carbonsäure, pharmazeutisch annehmbaren Salzen davon und unter physiologischen Bedingungen spaltbaren Estern davon gemäss Anspruch 1.

14. Verfahren zur Herstellung von (5R,6S)-2-[(2S)-Alanylaminomethyl]-6-[(1R)-1-hydroxyäthyl]-2-penem-3-carbonsäure, pharmazeutisch annehmbaren Salzen davon und unter physiologischen Bedingungen spaltbaren Estern davon gemäss Anspruch 1.

15. Verfahren zur Herstellung von (5R,6S)-2-[(2S)-Tyrosylaminomethyl]-6-[(1R)-1-hydroxyäthyl]-2-penem-3-carbonsäure, pharmazeutisch annehmbaren Salzen davon und unter physiologischen Bedingungen spaltbaren Estern davon gemäss Anspruch 1.

16. Verfahren zur Herstellung von (5R,6S)-2-[(2S)-Threonylaminomethyl]-6-[(1R)-1-hydroxyäthyl]-2-penem-3-carbonsäure, pharmazeutisch annehmbaren Salzen davon und unter physiologischen Bedingungen spaltbaren Estern davon gemäss Anspruch 1.

17. Verfahren zur Herstellung von (5R,6S)-2-[(2S)-Serylaminomethyl]-6-[(1R)-1-hydroxyäthyl]-2-penem-3-carbonsäure, pharmazeutisch annehmbaren Salzen davon und unter physiologischen Bedingungen spaltbaren Estern davon gemäss Anspruch 1.

18. Verfahren zur Herstellung von (5R,6S)-2-[(2R)-Serylaminomethyl]-6-[(1R)-1-hydroxyäthyl]-2-penem-3-carbonsäure, pharmazeutisch annehmbaren Salzen davon und unter physiologischen Bedingungen spaltbaren Estern davon gemäss Anspruch 1.

19. Verfahren zur Herstellung von pharmazeutischen Präparaten, dadurch gekennzeichnet, dass man eine gemäss Anspruch 1 erhältliche Verbindung der Formel I oder ein pharmazeutisch annehmbares Salz davon mit einem pharmazeutisch annehmbaren Trägerstoff mischt.

FO 7.4/UL/jt*/gs*/cs*

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0201459

Nummer der Anmeldung

EP 86 81 0193

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A- 0 125 208 (CIBA-GEIGY)<br>* Ansprüche *<br>--- | 1,19,<br>23 | C 07 D 499/00<br>A 61 K 31/43//<br>C 07 D 205/08<br>C 07 F 7/18<br>C 07 F 9/65 |
| A | EP-A- 0 070 204 (SUMITOMO)<br>* Zusammenfassung; Ansprüche 1-13 *<br>------- | 1,19,<br>23 | |
| | | | **RECHERCHIERTE SACHGEBIETE (Int. Cl.4)** |
| | | | C 07 D 499/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-21,23,24

Unvollständig recherchierte Patentansprüche:

Nicht recherchierte Patentansprüche: 22

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 12-08-1986 | CHOULY |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument